# EUROPEAN PATENT APPLICATION

(11) **EP 4 775 668 A2**
(43) Date of publication of application: **15.07.2026**
(21) Application number: 26181023.8
(22) Date of filing: 23.01.2020
(51) Int. Cl.: C12N 5/0781

(54) **B CELL IMMUNOTHERAPY**

(30) Priority: 23.01.2019 US 201962795629 P; 24.04.2019 US 201962837765 P
(62) Divisional of application: 20745872.0
(71) Applicant: The General Hospital Corporation, Boston, MA 02114 (US); Holy Cross Hospital, Inc., Fort Lauderdale, FL 33308 (US)
(72) Inventor: POZNANSKY, Mark, C., Boston, 02114 (US); SIRBULESCU, Ruxandra, F., Boston, 02114 (US)
(74) Representative: Weickmann & Weickmann PartmbB

(57) **Abstract**

The invention, in general, features a method of treating a neurodegenerative disease (such as amyotrophic lateral sclerosis) or a traumatic brain injury in a subject (e.g., a human) in need thereof, the method comprising administering to the subject a therapeutically effective amount of isolated B cells (such as autologous or allogeneic or xenogeneic B cells).

## Description

### CROSS-REFERENCE TO RELATED APPLICATIONS

This application claims benefit of U.S. Provisional Application No. 62/798,629, filed January 23, 2019, U.S. Provisional Application No. 62/837,765, filed April 24, 2019, and U.S. Provisional Application No. 62/965,032, filed January 23, 2020.

### BACKGROUND OF THE INVENTION

Degenerative diseases are medical conditions causing cells, tissues, or organs to deteriorate. Amyotrophic lateral sclerosis (ALS), Parkinson's disease (PD), Alzheimer's disease (AD), and Huntington's disease (HD), for example, are among many neurodegenerative diseases that involve degeneration of regions of the central nervous system. Traumatic brain injury (TBI) is also an example of a disorder which might increase the risk of a developing a degenerative brain disease such as PD or AD. Rheumatoid arthritis and osteoarthritis are still other examples of degenerative diseases involving inflammation. For most of these, there is no effective treatment available. Treatments for some of these diseases or disorders are under investigation. Proposed treatments, however, are very often expensive and involve significant risks and complications. There is accordingly a need for better approaches for treatment of degenerative diseases including neurodegenerative disease such as ALS and TBI.

### SUMMARY OF THE INVENTION

Provided herein are compositions including B cells (e.g., isolated, purified, or modified B cells or a combination thereof) and uses thereof for the treatment of disease (e.g., neurodegenerative diseases, traumatic brain injury (TBI), spinal cord injury (SCI), and inflammatory and immune diseases as described herein).

In a first aspect, the invention features a method of treating a neurodegenerative disease in a subject in need thereof, the method including administering to the subject a therapeutically effective amount of isolated B cells.

In some embodiments, the neurodegenerative disease is selected from amyotrophic lateral sclerosis (ALS), Parkinson's disease, Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease. In a preferred embodiment, the neurodegenerative disease is ALS (e.g., sporadic or familial ALS). In yet another preferred embodiment, the neurodegenerative disease is Parkinson's Disease.

In another aspect, the invention features a method of treating a subject having ALS, including administering to the subject a therapeutically effective amount of B cells, wherein the therapeutically effective amount is an amount sufficient to reduce or ameliorate one or more symptoms of ALS.

In another aspect, the invention features a method of treating a subject having ALS who exhibits one or more symptoms of ALS, including administering to the subject a therapeutically effective amount of B cells, wherein the therapeutically effective amount is an amount that results in reduction or amelioration of one or more of the symptoms of ALS; monitoring one or more of the symptoms in the subject; and administering a second dose of B cells when the one or more symptoms begins to worsen.

In some embodiments, the one or more symptoms of ALS include difficulty lifting the front part of the foot; difficulty lifting the toes; weakness in one or both legs; weakness in one or both feet; weakness in one or both ankles; hand weakness; hand clumsiness; muscle cramps; fasciculations (muscle twitches) in one or both arms, in one or both legs, in one or both shoulders, or of the tongue; muscle cramps; spasticity (tight and stiff muscles); and difficulty chewing or swallowing (dysphagia), difficulty speaking or forming words (dysarthria), and difficulty breathing (dyspnea).

In some embodiments, the method includes monitoring the one or more symptoms of ALS between 1 and 7 days post-administration, between 7 and 28 days post administration, between 1 and 28 weeks post-administration, between 1 and 2 months post-administration, between 2 and 6 months post-administration, between 2 and 9 months post-administration, or between 6 months and a year or more post-administration.

In some embodiments, the invention features a method for monitoring the responsiveness of a patient having a neurodegenerative disease to treatment with a therapeutically effective amount of isolated B cells by determining the level of a molecular marker of disease progression (e.g., determining the level of a molecular marker of neurodegenerative diseases progression before and after treatment with a therapeutically effective amount of B cells). The level of a molecular marker may be determined according to methods known to those of skill in the art.

In some embodiments, the level of a molecular marker may be determined from a sample from the treated subject, e.g., from a sample of blood plasma or cerebrospinal fluid (CSF) of a treated subject. Exemplary molecular markers of the progression of neurodegenerative diseases described herein are known in the art. Exemplary markers include T-tau (total tau), P-tau (hyperphosphorylated tau), Aβ42 (amyloid beta 42), the ratio of Aβ42/Aβ40, YKL-40 (Chitinase-3-like protein 1), VLP-1 (visinin-like protein 1), NFL (neurofilament light), pNFH (phosphorylated neurofilament heavy subunit), Ng (neurogranin) and UCH-L1 (ubiquitin C-terminal hydrolase), TDP-43 (TAR DNA-binding protein 43), decreased α-synuclein and/or decreased levels of 3,4-dihydroxyphenylacetate (see, e.g., Robey and Panegyres. Cerebrospinal fluid biomarkers in neurodegenerative disorders. Future Neurol. 14(1). (2019)).

In another aspect, the invention features a method of treating an inflammatory or immune disease in a subject in need thereof, the method including administering to the subject a therapeutically effective amount of isolated B cells.

In some embodiments, the inflammatory or immune disease is selected from cystic fibrosis, cardiovascular disease (e.g., coronary artery disease or aortic stenosis), keratoconus, keratoglobus, osteoarthritis, osteoporosis, pulmonary arterial hypertension, retinitis pigmentosa, and rheumatoid arthritis.

In another aspect, the invention features a method of treating a subject having a central nervous system (CNS) injury, including administering to the subject a therapeutically effective amount of isolated B cells. In some embodiments, the CNS injury is a traumatic brain injury (TBI) or a spinal cord injury (SCI). In some embodiments, the CNS injury includes both TBI and SCI.

In another aspect, the invention features a method of treating a subject having a traumatic brain injury (TBI), including administering to the subject a therapeutically effective amount of isolated B cells.

In another aspect, the invention features a method of treating a subject having a spinal cord injury (SCI), including administering to the subject a therapeutically effective amount of isolated B cells.

In some embodiments, the TBI is damage to the brain resulting from external mechanical force. In some embodiments, the SCI involves damage to the spinal cord resulting from external mechanical force. In some embodiments, the TBI and/or SCI results from a head injury or a cerebral contusion (e.g., resulting from a fall, a firearm wound, a sports accident, a construction accident, a vehicle accident, or an injury which penetrates the skull or brain of the subject). In some embodiments, the subject having TBI and/or SCI suffers from one or more of a number of physical, cognitive, social, emotional and/or behavioral disorders. TBI and SCI may be co-occurring and may result from the same injury.

In another aspect, the invention features a method of treating a subject having TBI who exhibits one or more symptoms of TBI, including administering to the subject a therapeutically effective amount of B cells, wherein the therapeutically effective amount is an amount that results in reduction or amelioration of one or more of the symptoms of TBI; monitoring one or more of the symptoms in the subject; and administering a second dose of B cells when the one or more symptoms begins to worsen.

In another aspect, the invention features a method of treating a subject having SCI (who exhibits one or more symptoms of SCI) including administering to the subject a therapeutically effective amount of B cells, wherein the therapeutically effective amount is an amount that results in reduction or amelioration of one or more of the symptoms of SCI; monitoring one or more of the symptoms in the subject; and administering a second dose of B cells when the one or more symptoms begins to worsen.

In some embodiments, the one or more symptoms of TBI and/or SCI include an inability to recall the traumatic event, confusion, difficulty learning and remembering new information, affective and executive dysfunction, trouble speaking coherently, unsteadiness, lack of coordination, and problems with vision or hearing; cognitive problems (e.g., amnesia, inability to speak or understand language, mental confusion, difficulty concentrating, difficulty thinking and understanding, inability to create new memories, or inability to recognize common things); behavioral problems (e.g., abnormal laughing and crying, aggression, impulsivity, irritability, lack of restraint (impulsiveness), or persistent repetition of words or actions); mood problems (e.g., anger, anxiety, apathy, or loneliness); whole body problems (e.g., blackout, dizziness, fainting, or fatigue); eye problems (e.g., dilated pupil, raccoon eyes, or unequal pupils); muscular problems (e.g., instability or stiff muscles); gastrointestinal problems (e.g., nausea or vomiting); speech problems (e.g., difficulty speaking or slurred speech); visual problems (e.g., blurred vision or sensitivity to light); bruising, depression, loss of smell, nerve injury, post-traumatic seizure, ringing in the ears, sensitivity to sound, vertigo.

In some embodiments, the method includes monitoring the one or more symptoms of TBI and/or SCI between 1 and 7 days post-administration, between 7 and 28 days post administration, between 1 and 28 weeks post-administration, between 1 and 2 months post-administration, between 2 and 6 months post-administration, between 2 and 9 months post-administration, or between 6 months and a year or more post-administration.

In some embodiments, the invention features a method for monitoring the responsiveness of a patient having TBI and/or SCI to treatment with a therapeutically effective amount of isolated B cells by determining the level of a molecular marker of disease progression (e.g., determining the level of a molecular marker of neurodegenerative diseases progression before and after treatment with a therapeutically effective amount of B cells). The level of a molecular marker may be determined according to methods known to those of skill in the art.

In some embodiments, the level of a molecular marker of TBI and/or SCI may be determined from a sample for the treated subject, e.g., from a sample of blood plasma or cerebrospinal fluid (CSF) of a treated subject. Exemplary molecular markers of the progression of TBI described herein include, without limitation, protein biomarkers for neuronal cell body injury (UCH-L1, NSE), astroglial injury (GFAP, S100B), neuronal cell death (all-spectrin breakdown products), axonal injury (NF proteins), white matter injury (MBP), post-injury neurodegeneration (total Tau and phospho-Tau), post-injury autoimmune response (brain antigen-targeting autoantibodies) (see, e.g., Wang et al. An update on diagnostic and prognostic biomarkers for traumatic brain injury. Expert Rev Mol Diagn. 18(2): 165-180 (2018)).

In some embodiments, allogeneic B cells are administered. In some embodiments, the allogeneic B cells are haploidentical allogeneic B cells, HLA-matched allogenic B cells, or genetically-modified B cells (e.g., B cells that have been genetically modified, for example by CRISPR, to reduce the immunogenicity of the B cell).

In some embodiments, autologous B cells are administered.

In some embodiments, xenogeneic B cells are administered.

In some embodiments, the method includes administering a second therapeutic composition.

In some embodiments, wherein the disease or disorder is ALS, the second therapeutic composition is Edaravone, Riluzole, or an immunomodulatory composition (e.g., an anti-CD14 antibody, an anti-CDL40 antibody, or a composition including T_{reg} cells).

In some embodiments, wherein the disease or disorder is TBI and/or SCI, the second therapeutic composition is an antibiotic or a corticosteroid (e.g., prednisone).

In some embodiments, the B cells are mature naïve B cells.

In some embodiments, the B cells are stimulated ex vivo.

In some embodiments, the B cells are stimulated ex vivo with a Toll-like receptor (TLR) agonist.

In some embodiments, the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-lgG complex.

In some embodiments, the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipid, a glycolipid, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines (e.g., imiquimod, resiquimod, loxoribine, bropirimine), an antiviral compound, an unmethylated CpG oligodeoxynucleotide, and profilin.

In some embodiments, the B cells are stimulated ex vivo with an immunomodulatory cytokine (e.g., a pro-inflammatory cytokine, such as a pro-inflammatory cytokine selected from IL-1β, IL-2, IL-4, IL-6, TNFα, or IFNγ).

In some embodiments, the B cells are B_{reg} cells. In some embodiments, the B_{reg} cells express immunomodulatory cytokine IL-10. In some embodiments, the B_{reg} cells further express one or more additional immunomodulatory cytokines selected from IL-2, IL-4, IL-6, IL-35, TNF-α, TGFβ, PD-L1 FasL, and TIM1. In some embodiments, the B_{reg} cells express one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD. In some embodiments, the B_{reg} cells express B220, CD19, CD20, CD24, CD138, IgM, and IgD. In some embodiments, the B_{reg} cells express CD25 and CD71. In some embodiments, the B_{reg} cells do not express CD73. In some embodiments, the B_{reg} cells include at least 80% (e.g., at least 85%, 90%, 95%, or 98%) CD19+ B cells. In some embodiments, the B_{reg} cells include less than 10% (e.g., less than 5%) CD138+ plasma B cells.

In some embodiments, the B cells are neuroprotective, anti-inflammatory, and/or immunomodulatory.

In some embodiments, the B cells are formulated to be administered locally or systemically. In some embodiments, the B cells are formulated to be administered intravenously, intraarterially, subcutaneously, intrathecally, or intraparenchymally. In some embodiments, the B cells are formulated to be administered by intravenous infusion or intravenous bolus. In some embodiments, the B cells are formulated to be administered through an intracranial cranial pressure (ICP) monitoring catheter.

In some embodiments, the B cells are administered once daily, once weekly, twice weekly, once every 14 days, once monthly, once every two months, once every three months, once every four months, once every five months, once every six months, or once yearly.

In some embodiments, the B cells are administered at least twice, three times, four times, five times, six times, seven times, eight times, nine times, or ten times.

In some embodiments, the therapeutically effective amount of B cells includes at least 0.5 X 10⁶ B cells per administration, 0.5 X 10⁷ B cells per administration, 1 x 10⁸ B cells per administration, at least 2 x 10⁸ B cells per administration, or at least 1 x 10⁹ B cells per administration. In some embodiments, the therapeutically effective amount of B cells includes 1 x 10⁸ B cells to 1 x 10⁹ B cells per administration, 1 x 10⁸ B cells to 5 x 10⁸ B per administration, or 2 x 10⁸ B cells to 4 x 10⁸ B cells per administration.

In another aspect, the invention features, a pharmaceutical composition including a modified B cell and one or more pharmaceutically-acceptable excipients, wherein the modified B cell has been stimulated ex vivo with a Toll-like receptor (TLR) agonist and/or an immunomodulatory cytokine.

In some embodiments, the modified B cell is a primary cell.

In some embodiments, the pharmaceutically-acceptable excipient is an aqueous solution (e.g., a saline solution).

In another aspect, the invention features, a method of treating a disease or condition in a subject in need thereof, the method including administering to the subject a pharmaceutical composition including a modified B cell and one or more pharmaceutically-acceptable excipients, wherein the modified B cell has been stimulated ex vivo with a Toll-like receptor (TLR) agonist and/or an immunomodulatory cytokine.

In some embodiments, the disease or condition is selected from abnormal wound healing (e.g., diabetic wound healing), a neurodegenerative disease, TBI, or SCI. Or an immune or inflammatory disease.

In some embodiments, the neurodegenerative disease is selected from amyotrophic lateral sclerosis (ALS), Parkinson's disease, Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease.

In some embodiments, the immune or inflammatory disease is selected from cystic fibrosis, cardiovascular disease (e.g., coronary artery disease or aortic stenosis), keratoconus, keratoglobus, osteoarthritis, osteoporosis, pulmonary arterial hypertension, retinitis pigmentosa, and rheumatoid arthritis.

In some embodiments, the modified B cell is an allogeneic B cell. In some embodiments, the allogeneic B cells is a haploidentical allogeneic B cell, an HLA-matched allogenic B cell, or a genetically-modified B cell (e.g., a B cell that has been genetically modified, for example by CRISPR, to reduce the immunogenicity of the B cell).

In some embodiments, modified B cell is an autologous B cell.

In some embodiments, modified B cell is a xenogeneic B cell.

In another aspect, the invention features, a method of producing a modified B cell, the method including:
i) isolating a mature naïve B cell from a subject, and
ii) stimulating the B cell ex vivo with a Toll-like receptor (TLR) agonist and/or an immunomodulatory cytokine,
thereby producing a modified B cell.

In some embodiments, step i) further includes isolating a CD19+ mature naïve B cell. In some embodiments, isolating the CD19+ mature naïve B cell is performed by immunoprecipitation with a CD19 antibody or antigen-binding fragment thereof. In some embodiments, the CD19 antibody or antigen-binding fragment thereof remains bound to the modified the modified B cell.

In some embodiments, the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-lgG complex.

In some embodiments, the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipids, a glycolipids, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines (e.g., imiquimod, resiquimod, loxoribine, bropirimine), an antiviral compound, an unmethylated CpG oligodeoxynucleotide, and profilin.

In some embodiments, the immunomodulatory cytokine is a pro-inflammatory cytokine (e.g., a pro-inflammatory cytokine selected from IL-1β, IL-2, IL-4, IL-6, TNFα, or IFNγ).

In some embodiments, the modified B cell is a B_{reg} cell. In some embodiments, the B_{reg} cell expresses immunomodulatory cytokine IL-10. In some embodiments, the B_{reg} cell further expresses one or more additional immunomodulatory cytokines selected from IL-2, IL-4, IL-6, IL-35, TNF-α, TGFβ, PD-L1 FasL, and TIM1. In some embodiments, the B_{reg} cell expresses one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD. In some embodiments, the B_{reg} cell expresses B220, CD19, CD20, CD24, CD138, IgM, and IgD. In some embodiments, the B_{reg} cell expresses CD25 and CD71. In some embodiments, the B_{reg} cell does not express CD73.

In some embodiments, the modified B cell is neuroprotective, anti-inflammatory, and/or immunomodulatory.

In still other embodiments, the invention features B cells which can be used directly as an anti-inflammatory and pro-regenerative cell-based therapeutic agent in a variety of disease contexts, including skin wounds and ulcers, muscular and cardiac injuries, brain and spinal cord injury, and lesions of various internal organs. Genetically-modified autologous, allogeneic or xenogeneic cells or cells primed with factors from an injured microenvironment are useful as having increased pro-regenerative efficiency. Factors derived from the B cells under these unique conditions, including antibodies, cytokines, and growth factors, as well as microRNA and other small molecules, may also be purified and applied directly to an injured tissue to accelerate healing.

B cells accordingly may be used as a therapeutic strategy for patients with neural degenerative diseases, TBI or SCI (e.g., resulting from a cerebral contusion), inflammatory disorders, or a variety of immune diseases. Unlike any other existing cell-based therapies, B cells can be readily obtained from peripheral blood or other blood bank products, an important advantage for the development of a rapid off-the-shelf therapeutic agent. Indeed, a rapid, minimally-manipulated, B cell therapy (allogeneic or autologous, or xenogeneic) is highly translatable into a clinical setting. This is especially true in not only treating neurodegenerative diseases such as ALS and PD but also in the case of severe brain lesions, where surgery is often performed to remove hematomas or penetrating bone fragments, and either intraparenchymal or intraventricular catheters are placed to monitor intracranial pressure, thus providing a convenient route of administration for B cells into injured brain.

Unlike other cell types used therapeutically, such as stem cells, B lymphocytes are mature, terminally-differentiated cells, with a naturally limited lifespan of 5-6 weeks in vivo. Their application in a neurodegenerative setting as well as in the disrupted microenvironment of a brain contusion is expected to lead to elimination of the transplanted cells after even less time. This is advantageous because longer survival of transplanted cells could represent a significant safety concern, particularly considering that the microenvironment of the central nervous system contains a number of B cell-trophic factors.

Our results illustrate a first proof-of-principle observation that mature peripherally isolated B cells represent a safe, rapid, and effective cell-based therapeutic strategy for several disorders disclosed herein (including ALS, PD, TBI, and SCI), for which no therapeutic options to improve neurological outcome exist currently.

Other objects, features and advantages of the present invention will become apparent from the following detailed description. It should be understood, however, that the detailed description and specific examples, while indicating preferred embodiments of the invention, are given by way of illustration only, since various changes and modifications within the scope and spirit of the invention will become apparent to one skilled in the art from this detailed description.

Some embodiments of the technology and methodologies described herein are defined according to any of the following numbered paragraphs.
1. A method of treating a neurodegenerative disease in a subject in need thereof, the method comprising administering to the subject a therapeutically effective amount of isolated B cells.
2. The method of paragraph 1, wherein the neurodegenerative disease is selected from amyotrophic lateral sclerosis (ALS), Parkinson's disease, Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease.
3. The method of paragraph 1, wherein allogeneic B cells are administered.
4. The method of paragraph 1, wherein autologous B cells are administered.
5. The method of paragraph 1, further comprising administering a second therapeutic composition.
6. The method of paragraph 5, wherein the second therapeutic composition is Edaravone or Riluzole.
7. The method of paragraph 6, wherein the second therapeutic composition is an immunomodulatory composition.
8. The method of any of paragraphs 1-7, wherein the B cells are mature naïve B cells.
9. The method of any of paragraphs 1-8, wherein B cells are stimulated ex vivo.
10. The method of any one of paragraphs 1-9, wherein the B cells are stimulated with a Toll-like receptor (TLR) agonist.
11. The method of paragraph 10, wherein the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-IgG complex.
12. The method of paragraph 10, wherein the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipids, a glycolipids, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines, an antiviral compound, an unmethylated CpG oligodeoxynucleotide, and profilin.
13. The method of any of paragraphs 1-12, wherein the B cells are B_{reg} cells.
14. The method of paragraph 13, wherein the B_{reg} cells express immunomodulatory cytokine IL-10.
15. The method of paragraph 14, wherein the B_{reg} cells further express one or more additional immunomodulatory cytokines selected from IL-2, IL-4, IL-6, IL-35, TNF-α, TGFβ, PD-L1 FasL, and TIM1.
16. The method of any one of paragraphs 13-15, wherein the B_{reg} cells express one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD.
17. The method of paragraph 16, wherein the B_{reg} cells express B220, CD19, CD20, CD24, CD138, IgM, and IgD.
18. The method of paragraph 16, wherein the B_{reg} cells express CD25 and CD71.
19. The method of any one of paragraphs 13-18, wherein the B_{reg} cells do not express CD73.
20. The method of any one of paragraphs 13-15, wherein the B_{reg} cells comprise at least 80% CD19+ B cells.
21. The method of any one of paragraph 13-15 or 20, wherein the B_{reg} cells comprise less than 10% CD138+ plasma B cells.
22. The method of any one of paragraphs 1-21, wherein the B cells are neuroprotective.
23. The method of any one of paragraphs 1-22, wherein the B cells are anti-inflammatory.
24. The method of any one of paragraphs 1-23, wherein the B cells are immunomodulatory.
25. The method of any of one of paragraphs 1-24, wherein the B cells are formulated to be administered locally.
26. The method of any one of paragraphs 1-24, wherein the B cells are formulated to be administered systemically.
27. The method of any one of paragraphs 1-24, wherein the B cells are formulated to be administered intravenously, intraarterially, subcutaneously, intrathecally, or intraparenchymally.
28. The method of paragraph 27, wherein the B cells are formulated to be administered by intravenous infusion or intravenous bolus.
29. The method of any one of paragraphs 1-28, wherein the B cells are administered once daily, once weekly, twice weekly, once every 14 days, once monthly, once every two months, once every three months, once every four months, once every five months, once every six months, or once yearly.
30. The method of any one of paragraphs 1-29, wherein the B cells are administered at least twice, three times, four times, five times, six times, seven times, eight times, nine times, or ten times.
31. The method of any of paragraphs 1-30, wherein the therapeutically effective amount comprises at least 1 x 10⁸ B cells per administration.
32. The method of any of paragraphs 1-30, wherein the therapeutically effective amount comprises at least 2 x 10⁸ B cells per administration.
33. The method of any of paragraphs 1-30, wherein the therapeutically effective amount comprises at least 1 x 10⁹ B cells per administration.
34. A method of treating a subject having a traumatic brain injury (TBI), comprising administering to the subject a therapeutically effective amount of isolated B cells.
35. The method of paragraph 34, wherein TBI results from a head injury, or a cerebral contusion.
36. The method of paragraph 34, wherein the subject suffers from one or more of a number of physical, cognitive, social, emotional and/or behavioral disorders.
37. The method of paragraph 34, wherein allogeneic B cells are administered.
38. The method of paragraph 34, wherein autologous B cells are administered.
39. The method of paragraph 34, additionally comprising administering a second therapeutic composition.
40. The method of paragraph 39, wherein the second therapeutic composition is an antibiotic or an corticosteroid.
41. The method of any of paragraphs 34-40, wherein the B cells are mature naïve B cells.
42. The method of any of paragraphs 34-41, wherein B cells are stimulated ex vivo.
43. The method of any one of paragraphs 34-42, wherein the B cells are stimulated with a Toll-like receptor (TLR) agonist.
44. The method of paragraph 43, wherein the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-IgG complex.
45. The method of paragraph 43, wherein the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipids, a glycolipids, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines, an antiviral compound, an unmethylated CpG oligodeoxynucleotide, and profilin.
46. The method of any of paragraphs 34-45, wherein the B cells are B_{reg} cells.
47. The method of paragraph 46, wherein the B_{reg} cells express immunomodulatory cytokine IL-10.
48. The method of paragraph 47, wherein the B_{reg} cells further express one or more additional immunomodulatory cytokines selected from IL-2, IL-4, IL-6, IL-35, TNF-α, TGFβ, PD-L1 FasL, and TIM1.
49. The method of any one of paragraphs 46-48, wherein the B_{reg} cells express one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD.
50. The method of paragraph 49, wherein the B_{reg} cells express B220, CD19, CD20, CD24, CD138, IgM, and IgD.
51. The method of paragraph 49, wherein the B_{reg} cells express CD25 and CD71.
52. The method of any one of paragraphs 46-51, wherein the B_{reg} cells do not express CD73.
53. The method of any one of paragraphs 46-48, wherein the B_{reg} cells comprise at least 80% CD19+ B cells.
54. The method of any one of paragraphs 46-48 or 53, wherein the B cells comprise less than 10% CD138+ plasma B cells.
55. The method of any one of paragraphs 34-54, wherein the B cells are neuroprotective.
56. The method of any one of paragraphs 34=55, wherein the B cells are anti-inflammatory.
57. The method of any one of paragraphs 34-56, wherein the B cells are immunomodulatory.
58. The method of any of one of paragraphs 34-57, wherein the B cells are formulated to be administered locally.
59. The method of any one of paragraphs 34-57, wherein the B cells are formulated to be administered systemically.
60. The method of any one of paragraphs 34-57, wherein the B cells are formulated to be administered intravenously, intraarterially, subcutaneously, intrathecally, or intraparenchymal.
61. The method of paragraph 60, wherein the B cells are formulated to be administered by intravenous infusion or intravenous bolus.
62. The method of any of paragraphs 34-57, wherein the B cells are formulated to be administered through an intracranial cranial pressure (ICP) monitoring catheter.
63. The method of any one of paragraphs 34-62, wherein the B cells are administered once daily, once weekly, twice weekly, once every 14 days, once monthly, once every two months, once every three months, once every four months, once every five months, once every six months, or once yearly.
64. The method of any one of paragraphs 34-63, wherein the B cells are administered at least twice, three times, four times, five times, six times, seven times, eight times, nine times, or ten times.
65. The method of any of paragraphs 34-63, wherein the therapeutically effective amount comprises at least 1 x 10⁸ B cells per administration.
66. The method of any of paragraphs 34-63, wherein the therapeutically effective amount comprises at least 2 x 10⁸ B cells per administration.
67. The method of any of paragraphs 34-63, wherein the therapeutically effective amount comprises at least 1 x 10⁹ B cells per administration.
68. A pharmaceutical composition comprising a modified B cell and one or more pharmaceutically-acceptable excipients, wherein the modified B cell has been stimulated ex vivo with a Toll-like receptor (TLR) agonist and/or an immunomodulatory cytokine.
69. The pharmaceutical composition of paragraph 68, wherein the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-lgG complex.
70. The pharmaceutical composition of paragraph 68, wherein the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipids, a glycolipids, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines, an antiviral compound, an unmethylated CpG oligodeoxynucleotide, and profilin.
71. The pharmaceutical composition of paragraph 68, wherein the immunomodulatory cytokine is a pro-inflammatory cytokine.
72. The pharmaceutical composition of paragraph 71, wherein the pro-inflammatory cytokine is selected from IL-1β, IL-2, IL-4, IL-6, TNFα, or IFNγ.
73. The pharmaceutical composition of any one of paragraphs 68-72, wherein the modified B cell is a primary cell.
74. The pharmaceutical composition of any of paragraphs 68-73, wherein the modified B cell is a B_{reg} cell**.**
75. The pharmaceutical composition of paragraph 74, wherein the B_{reg} cell expresses immunomodulatory cytokine IL-10.
76. The pharmaceutical composition of paragraph 75, wherein the B_{reg} cell further expresses one or more additional immunomodulatory cytokines selected from IL-2, IL-4, IL-6, IL-35, TNF-α, TGFβ, PD-L1 FasL, and TIM1.
77. The pharmaceutical composition of any one of paragraphs 74-76, wherein the B_{reg} cell expresses one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD.
78. The pharmaceutical composition of paragraph 77, wherein the B_{reg} cell expresses B220, CD19, CD20, CD24, CD138, IgM, and IgD.
79. The pharmaceutical composition of paragraph 77, wherein the B_{reg} cell expresses CD25 and CD71.
80. The pharmaceutical composition of any one of paragraphs 74-79, wherein the B_{reg} cell does not express CD73.
81. The pharmaceutical composition of any one of paragraphs 68-80, wherein the modified B cell is neuroprotective.
82. The pharmaceutical composition of any one of paragraphs 68-81, wherein the modified B cell is anti-inflammatory.
83. The pharmaceutical composition of any one of paragraphs 68-82, wherein the modified B cell is immunomodulatory.
84. The pharmaceutical composition of any one of paragraphs 68-83, wherein the pharmaceutically-acceptable excipient is an aqueous solution.
85. A method of treating a disease or condition in a subject in need thereof, the method comprising administering to the subject the pharmaceutical composition of any one of paragraphs 68-84.
86. The method of paragraph 85, wherein the disease or condition is abnormal wound healing.
87. The method of paragraph 85, wherein the disease or condition is a neurodegenerative disease.
88. The method of paragraph 87, wherein the neurodegenerative disease is selected from amyotrophic lateral sclerosis (ALS), Parkinson's disease, Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease.
89. The method of paragraph 85, wherein the disease or condition is traumatic brain injury (TBI).
90. The method of paragraph 85, wherein the disease or condition is selected from cystic fibrosis, cardiovascular disease, keratoconus, keratoglobus, osteoarthritis, osteoporosis, pulmonary arterial hypertension, retinitis pigmentosa, and rheumatoid arthritis.
91. The method of any one of paragraphs 85-90, wherein the modified B cell is an allogeneic B cell.
92. The method of any one of paragraphs 85-91, wherein the modified B cell is an autologous B cell.
93. A method of producing a modified B cell, the method comprising:
   i) isolating a mature naïve B cell from a subject, and
   ii) stimulating the B cell ex vivo with a Toll-like receptor (TLR) agonist and/or an immunomodulatory cytokine,
   thereby producing a modified B cell.
94. The method of paragraph 93, wherein step i) further comprises isolating a CD19+ mature naïve B cell.
95. The method of paragraph 94, wherein isolating the CD19+ mature naïve B cell is performed by immunoprecipitation with a CD19 antibody or antigen-binding fragment thereof.
96. The method of paragraph 95, wherein the CD19 antibody or antigen-binding fragment thereof remains bound to the modified the modified B cell.
97. The method of paragraph 93, wherein the TLR agonist is an endogenous ligand selected from a heat shock protein, a necrotic cell or a fragment thereof, an oxygen radical, a urate crystal, an mRNA, a beta-defensin, fibrin, fibrinogen, Gp96, Hsp22, Hsp60, Hsp70, HMGB1, lung surfactant protein A, low density lipoprotein (LDL), pancreatic elastase, a polysaccharide fragment of heparan sulfate, soluble hyaluronan, alpha A-crystallin, and a CpG chromatin-IgG complex.
98. The method of paragraph 93, wherein the TLR agonist is an exogenous ligand selected from Pam3CSK4, a triacylated lipopeptide, a glycosylphosphatidylinositol (GPI)-anchored protein, lipoarabinomannan, an outer surface lipoprotein, a lipopolysaccharide, a cytomegalovirus envelope protein, a glycoinositolphospholipids, a glycolipids, a GPI anchor, Herpes simplex virus 1 or a fragment thereof, lipoteichoic acid, a mannuronic acid polymer, a bacterial outer membrane porin, zymosan, double-stranded RNA, single-stranded RNA, Poly(I).Poly(C), taxol, flagellin, modulin, an imidazoquinolines, an antiviral compound, an unmethylated CpG oligodeoxynucleotide, and profilin.
99. The method of paragraph 93, wherein the immunomodulatory cytokine is a pro-inflammatory cytokine.
100. The method of paragraph 99, wherein the pro-inflammatory cytokine is selected from IL-1β, IL-2, IL-4, IL-6, TNFα, or IFNγ.
101. The method of any of paragraphs 93-100, wherein the modified B cell is a B_{reg} cell.
102. The method of paragraph 101, wherein the B_{reg} cell expresses immunomodulatory cytokine IL-10.
103. The method of paragraph 102, wherein the B_{reg} cell further expresses one or more additional immunomodulatory cytokines selected from IL-2, IL-4, IL-6, IL-35, TNF-α, TGFβ, PD-L1 FasL, and TIM1.
104. The method of any one of paragraphs 101-103, wherein the B_{reg} cell expresses one or more cell surface markers selected from B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD.
105. The method of paragraph 105, wherein the B_{reg} cell expresses B220, CD19, CD20, CD24, CD138, IgM, and IgD.
106. The method of paragraph 105, wherein the B_{reg} cell expresses CD25 and CD71.
107. The method of any one of paragraphs 101-106, wherein the B_{reg} cell does not express CD73.
108. The method of any one of paragraphs 93-107, wherein the modified B cell is neuroprotective.
109. The method of any one of paragraphs 93-108, wherein the modified B cell is anti-inflammatory.
110. The method of any one of paragraphs 93-109, wherein the modified B cell is immunomodulatory.

### BRIEF DESCRIPTION OF THE DRAWINGS

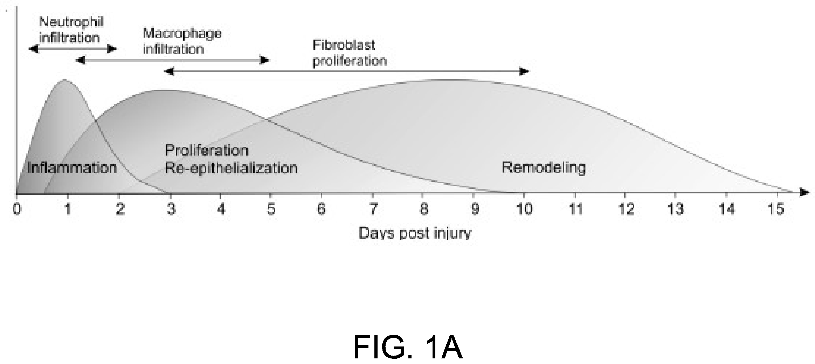

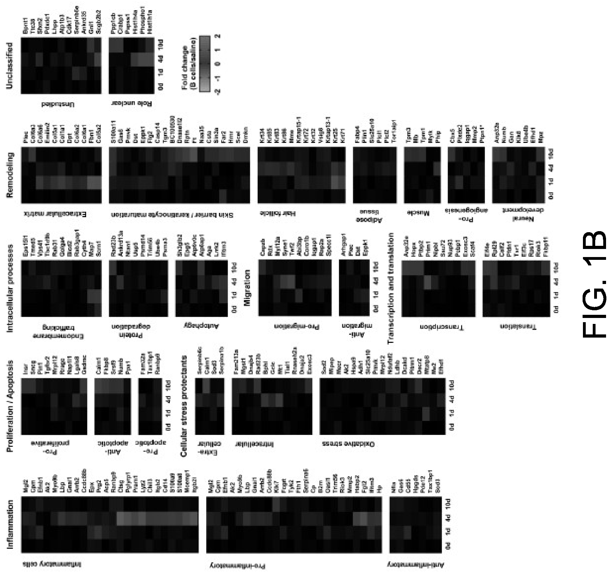

FIG.1A - FIG. 1B shows B cell application induces complex changes in the molecular microenvironment of a wound. FIG. 1A is a schematic representation of the average duration of the major stages of wound healing in the wild-type murine wound model. FIG. 1B shows heatmaps summarizing the expression dynamics over time in proteins with significantly altered expression in response to B cell application. A total of 213 proteins representing the aggregate of significantly altered proteins associated with B cell treatment (n = 111; p < 0.05, unpaired t-test) as well as those proteins with high fold change (top 20 up- or down-regulated proteins) for each time point, regardless of significance level (n = 112), were classified according to processes relevant to wound healing. Heatmaps show fold change expression after B cell treatment at 0, 1, 4, 10 d post-injury. Red = up-regulation; Green = down-regulation. Particularly notable were the down-regulation of multiple proteins associated with inflammation and inflammatory cells at 4 days post-injury and the substantial up-regulation of proteins associated with cell proliferation, protection from apoptosis (cell death) and oxidative stress, and tissue remodeling (formation of hair follicles and muscle) at 4-10 days post injury.

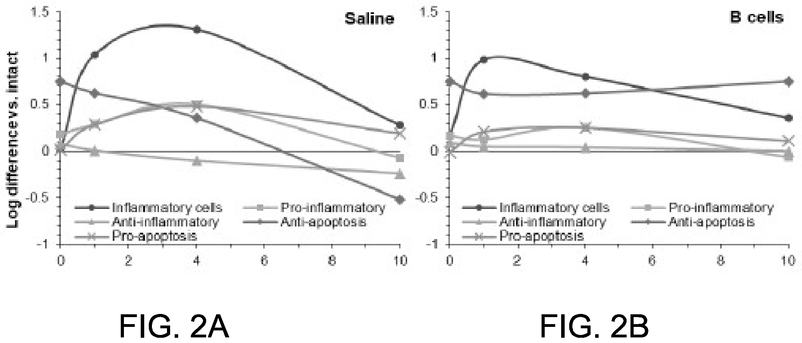

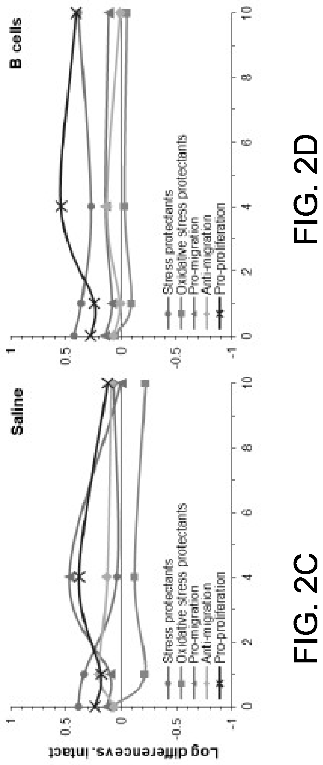

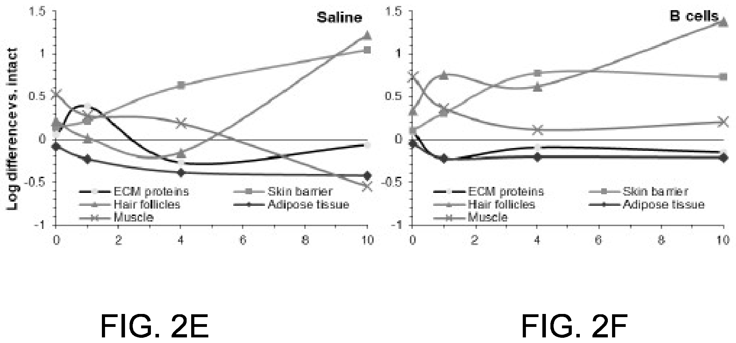

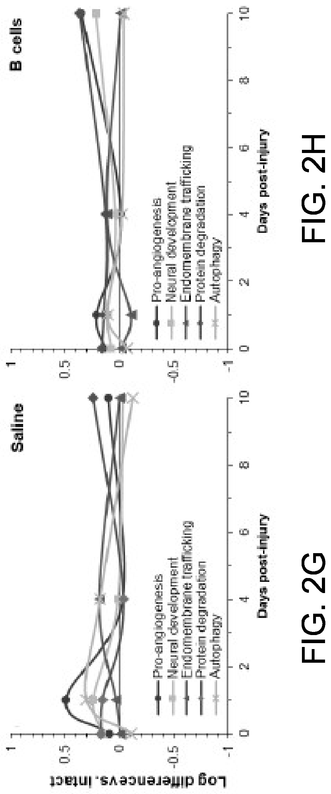

FIG. 2A - FIG. 2H show average expression of proteins by functional family over time in wounds treated with saline (controls, regular wound healing) or after B cell treatment. This analysis illustrates the overall effect of B cells as a homeostatic agent, rather than an inducer or inhibitor of protein expression B cell application was associated with the maintenance of steady levels of expression of proteins that normally either decline or increase during the course of injury and healing, significantly reducing the inflammatory peak observed in normal healing, preventing the reduction in anti-apoptotic factors (arrows) and oxidative stress protectants and increasing proliferation (FIG.2A - FIG.2B), reducing the decline in anti-oxidative stress protectants and in cell proliferation, and maintaining cell migration at lower levels (FIG. 2C - FIG. 2D), maintaining steady levels of proteins associated with remodeling and secondary skin structures (FIG. 2E - FIG. 2F), reducing levels of protein degradation and autophagy observed in the early stages of injury in controls, and increasing levels of proteins associated with angiogenesis and nerve regeneration at late stages of healing (FIG. 2G - FIG. 2H).

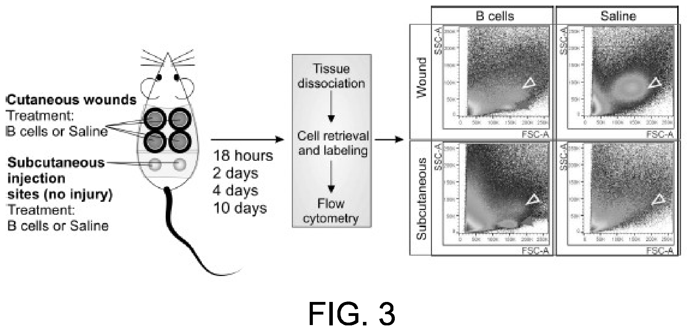

FIG. 3 shows the experimental paradigm for in vivo assessment of B cell application in acute wound healing. A total of 4 full-thickness lesions were generated in the dorsal skin of a wild-type C57BI6 mouse and mature naïve B cells purified from isogeneic animals were applied directly on the wound beds. Control animals received saline applications. As internal control, B cells or saline control were also injected subcutaneously under intact skin to provide a similar microenvironment without the injury. After defined survival times, the wound or cutaneous uninjured tissue was collected, dissociated, and processed for flow cytometry analysis. Scatterplots on the right show typical distributions of cell suspensions from each treatment category. Wound samples show a characteristic influx of leukocytes (white open arrowhead) that is largely absent in uninjured tissue. While few B cells are typically present at either location, they can easily be detected in large numbers after experimental application (red arrow).

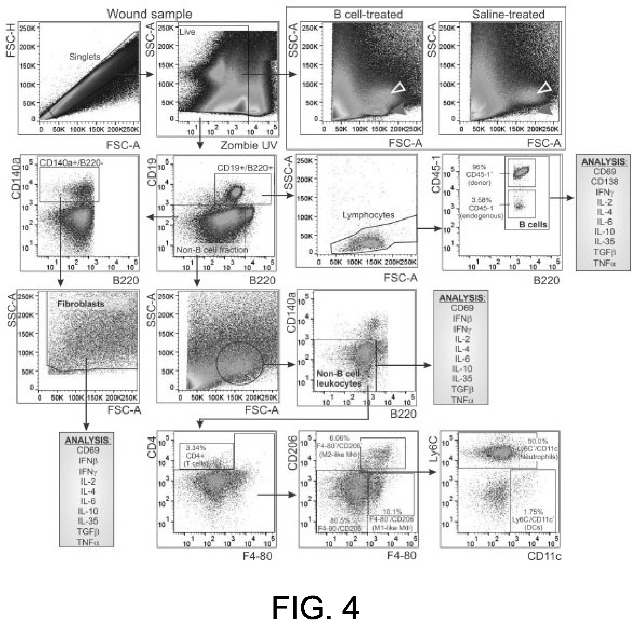

FIG. 4 shows the gating strategy and analysis of B cell-treated and control wound cell suspensions via flow cytometry. Live cells were gated into 3 main categories: B cells (CD19+/B220+ lymphocytes), non-B cell leukocytes (CD140a-/B220- leukocytes) which included a mix of neutrophils, monocytes and macrophages, dendritic cells and T cells, and fibroblasts (CD140a+/B220-). These cell categories were evaluated for markers of activation and cytokine production.

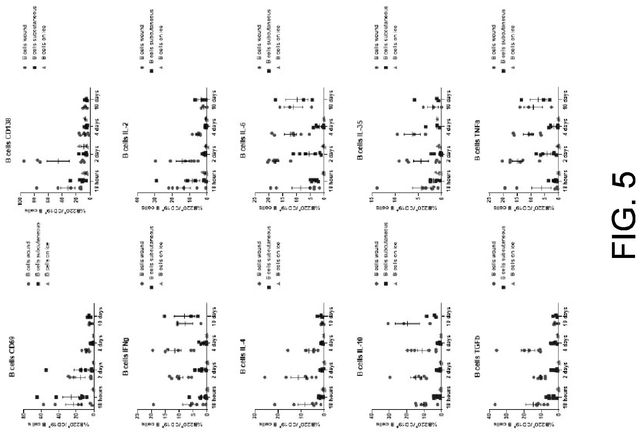

FIG. 5 shows the dynamics of activation markers and key cytokines in B cells retrieved from the wound bed after defined exposure intervals to the wound microenvironment. B cells were exposed in vivo to the wound niche or injected under uninjured skin (control equivalent location). Control B cells maintained on ice immediately after isolation for the same time duration are shown for comparison. After time intervals including 18 hours, 2 days, 4 days, and 10 days, the wounds were treated with Brefeldin A for 4 hours to induce retention of cytokines within cells. B cells were then retrieved by excising and dissociating the tissue, and further characterized by flow cytometry both for surface markers and intracellular cytokines. B cells exposed to the wound microenvironment transiently upregulate multiple immunomodulatory cytokines, peaking at 2 days post-application. Some immunomodulatory cytokines, including TGFβ and IL-6 remain elevated at 4 days, and IL-10 up to 10 days. N = 3-6 animals/group.

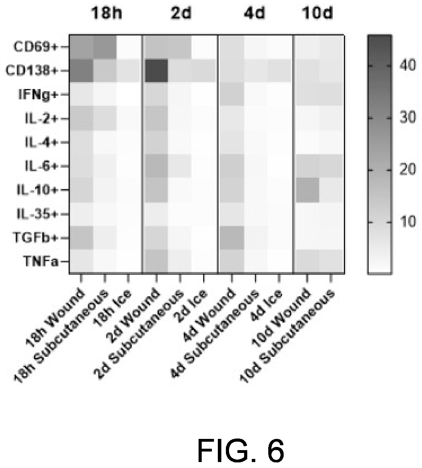

FIG. 6 is a heatmap summary of the average values for each marker in B cells exposed to the wound microenvironment, subcutaneous control, or maintained on ice (no exposure).

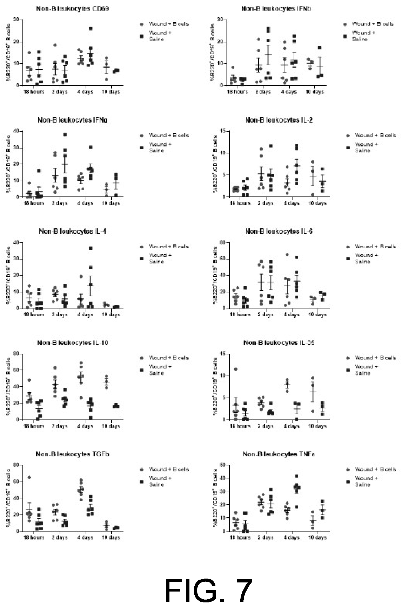

FIG. 7 shows the dynamics of activation markers and key cytokines in the aggregate infiltrating non-B cell leukocytes in the wound. Overall, infiltrating leukocytes produced more anti-inflammatory cytokines IL-10, TGFβ, and IL-35, and less pro-inflammatory TNFα and IL-2 when B cells were present in the wound. This impact was most pronounced at 4 days post injury and B cell application and persisted up to 10 days. N = 3-6 animals/group.

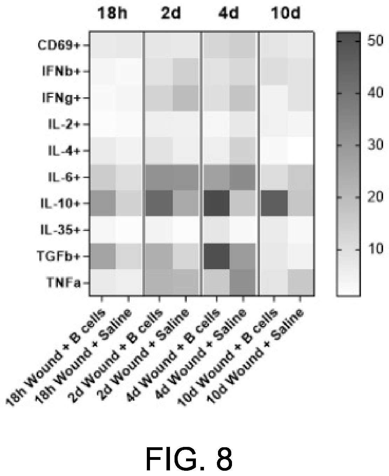

FIG. 8 is a heatmap summary of the average values for each marker in infiltrating non-B cell leukocytes in the wound microenvironment, illustrating the pattern of increased anti-inflammatory cytokine (IL-10 and TGFβ) production in the presence of B cells.

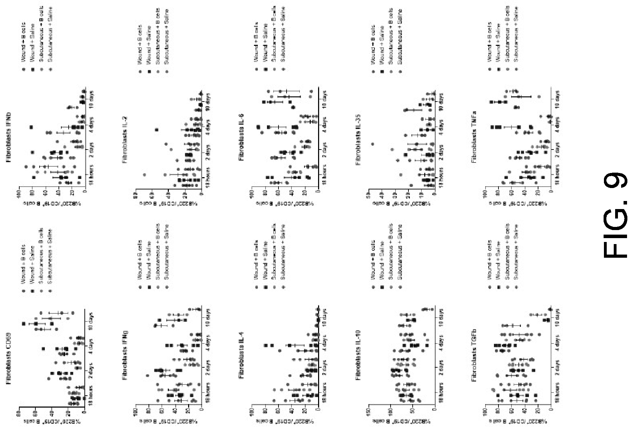

FIG. 9 shows the dynamics of activation markers and key cytokines in the CD140a+ fibroblast population of the wound and subcutaneous tissue. Fibroblasts in the wound produced significantly more IL-10 and TGFβ at 10 days post-injury when wounds were exposed to B cells. Moreover, wound fibroblasts produce less of the pro-inflammatory cytokine TNFα when B cells were applied, both at 4 days and 10 days post-injury.

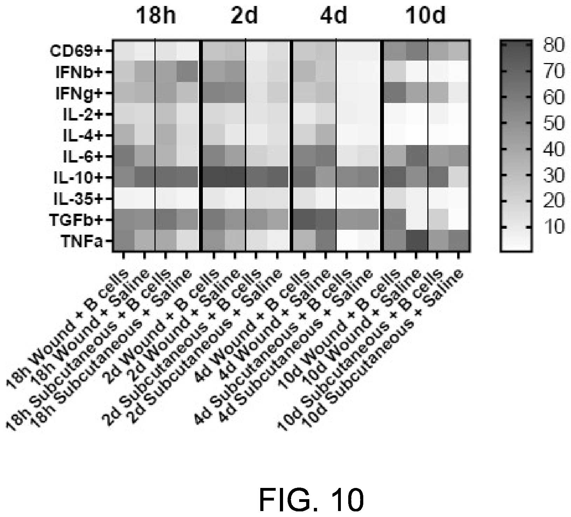

FIG. 10 is a heatmap summary of the average values for each marker in fibroblasts in wounds and subcutaneous tissue treated either with B cells or saline solution. Fibroblasts are among the most important sources of anti-inflammatory and pro-regenerative factors in wound healing and generate high levels of IL-10 and TGFβ regardless of treatment. Nevertheless, fibroblasts from wounds treated with B cells continued to produce higher levels of both IL-10 and TGFβ at 4 and 10 days post-injury, while in saline-treated wounds, the levels of these anti-inflammatory cytokines decreased. Interestingly, a significant effect of B cell application was observed in the reduction of pro-inflammatory cytokines in wound fibroblasts, including IL-6 and TNFα.

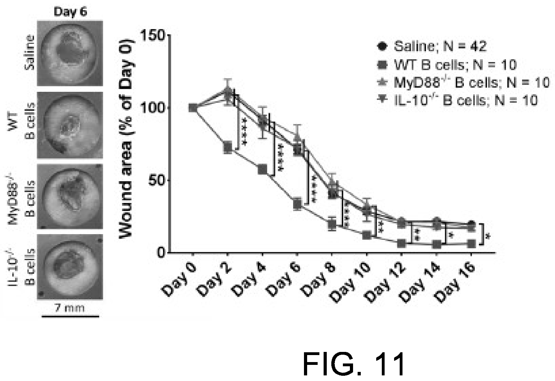

FIG. 11 shows functional TLR signaling as well as IL-10 production are necessary components of the regenerative function of exogenous B cells in wound healing. Full-thickness excision wounds (illustrated here at day 6 of healing) were treated at day 0 with B cells lacking the common TLR-signaling adaptor myeloid differentiation factor 88 (MyD88), IL-10, or WT B cells as control. Saline was also included as internal control in each tested animal. While the WT B cells consistently accelerated the wound closure by 2-3 days in WT animals, MyD88-/- or IL-10-/- B cells showed no benefit for wound closure, similar to saline application.

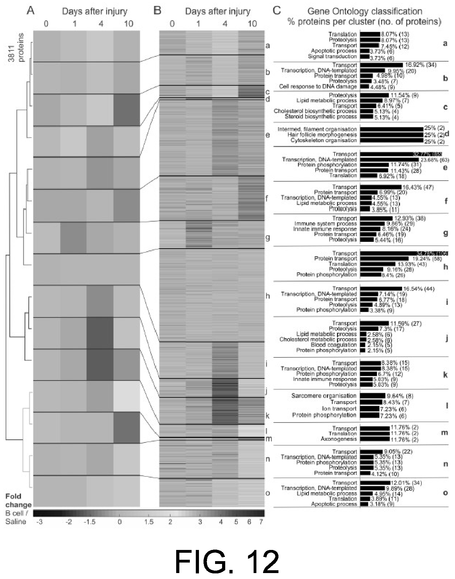

FIG. 12 shows an unsupervised hierarchical cluster analysis of identified proteins expressed in skin wound samples. Only identified proteins that were found consistently present across all samples were included in the analysis. (A) Hierarchical clustering using complete linkage of 3809 proteins (rows) consistently expressed in all animals in both B cell and saline treated wounds at 4 different time points after injury (columns). The pseudocolor scale depicts normalized, log-transformed fold change expression values for each protein. The dendrogram shows 15 protein clusters derived from this analysis, with the color of each cell in (A) mapped to the mean expression value of the cluster at the respective time points. Proteins cluster by their pattern of expression over time. (B) Heatmap of hierarchical cluster from (A) showing all 3809 proteins. (C) Gene ontology analysis of the 15 hierarchical clusters. The mouse GOslim gene list from QuickGO (accessible at https://www.ebi.ac.uk/QuickGO) was used to probe the 15 hierarchical clusters. Bar graphs show the top biological function categories for each cluster.

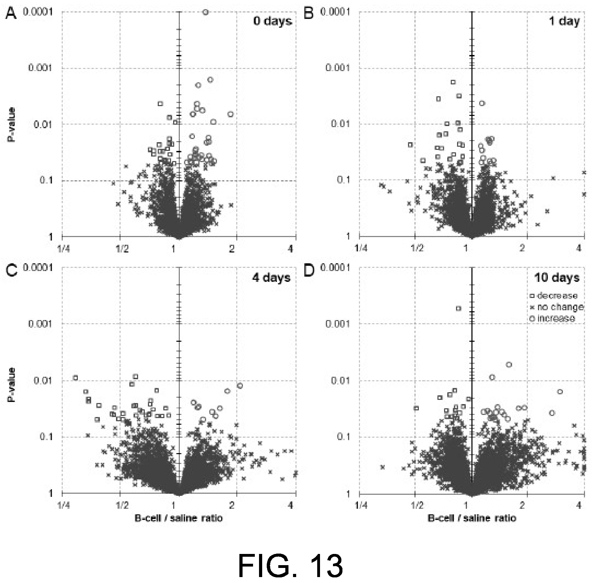

FIG. 13 shows the distribution of significantly altered proteins in response to B cell treatment at each assessed time point during wound healing.

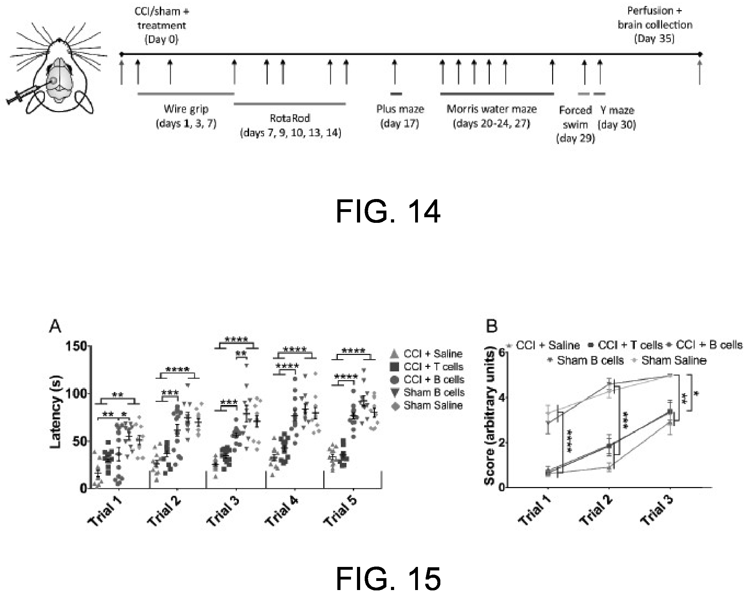

FIG. 14 shows experimental paradigm for assessing the effect of B cell application on functional (behavioral) and histological recovery after contusion TBI. Adult male C57BL/6J mice were anesthetized and a 5-mm circular craniotomy was performed above the left parieto-temporal cortex, and the bone flap was removed. A single infusion of 2×10⁶ B cells, was delivered intraparenchymally into the ipsilateral hemisphere just prior to injury. Mice were then subjected to CCI or sham injury. After recovery, motor function, motor and spatial learning and memory performance, anxiety, and depression-like behavior were assessed using multiple assays. At the end of the behavioral testing (Day 35), the animals were euthanized, and the brains were collected for evaluation of total lesion volume.
FIG. 15 shows effect of acute B cell treatment on vestibulo-motor function and striatal learning. (A) Rotarod assessment showed a significant protective effect of B cells administered at the time of CCI. Notably, the latency to fall over repeated trials increased in B cell treated mice as well as in sham-lesioned animals, suggesting a motor learning component. No such improvement was observed in controls treated with T cells or saline. After the second trial, no significant difference was observed between CCI injured mice that received B cell treatment and sham-lesioned B cell-treated animals. (B) Assessment of vestibulo-motor recovery after injury using the wire grip assay showed a significant effect of injury as compared to sham-lesioned controls, however no statistically significant difference was observed between the treatment conditions in the injured mice. Data are mean ± SEM. * p<0.05; ** p<0.01; *** p<0.001; **** p<0.0001. CCI + B cells, *n* = 12 mice; CCI + T cells, *n* = 12 mice; CCI + saline, *n* = 12 mice; sham-treated + B cells, *n* = 10 mice; sham-treated + saline, *n* = 10 mice.

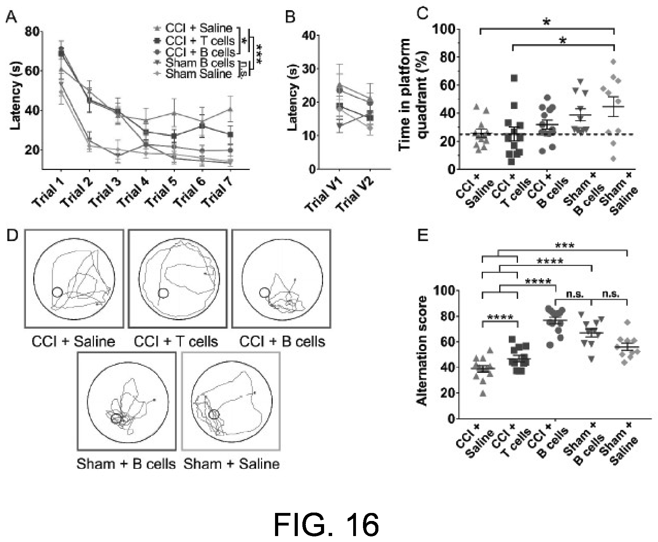

FIG. 16 shows effect of a single acute B cell application on learning and memory. (A-D) Morris water maze assessment. Learning curves showed a significant improvement in CCI mice treated with B cells over saline-treated CCI animals (p < 0.05). No significant difference was observed between B cell treated injured animals and either of the sham-lesioned conditions after the third trial (p >0.98) (A). Visible platform trials showed no difference between the treatment conditions with or without injury (B). (C) The probe trial showed that the B cell -treated CCI injured animals spent above-chance time in the target quadrant, not significantly different from sham-lesioned mice. By contrast, control CCI injured mice treated with either T cells or saline only spent chance-level time exploring the target quadrant and differed significantly from the sham-lesioned animals (p < 0.05). *Dashed line* indicates chance level. (D) Representative swim path tracings during the probe trial indicate spatial search patterns in CCI-B cell mice as wells as in both sham groups, whereas non-spatial strategies were employed by CCI mice in the T cell and saline groups. (E) Y maze assessment of short-term learning and memory. B cell treated CCI mice had significantly higher alternation scores than injured T cell- or saline-treated groups, performing similarly to sham groups. All data are shown as mean ± SEM. * p<0.05; ** p<0.01; *** p<0.001; **** p<0.0001. CCI + B cells, *n* = 12 mice; CCI + T cells, *n* = 12 mice; CCI + saline, *n* = 12 mice; sham-treated + B cells, *n* = 10 mice; sham-treated + saline, *n* = 10 mice.

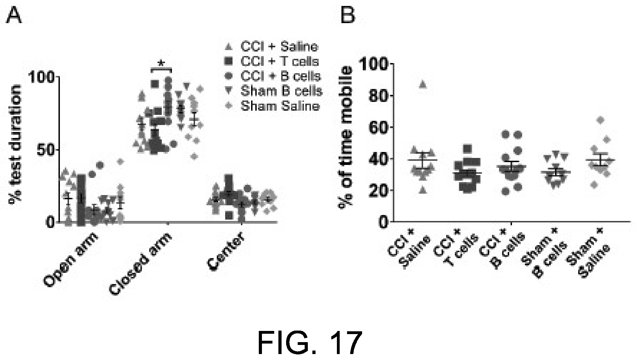

FIG. 17 shows effect of B cell treatment on anxiety and depression-like behavior after CCI. (A) Elevated plus maze assay of anxiety-like behavior. No overall significant differences were observed between treatment groups with the exception of a modest difference in the time spent in the closed arm between CCI injured mice that received B cells versus animals that received equal numbers of T cells at the time of injury (*p < 0.05). (B) Forced swim assay of depressive-like behavior. No effect of lesion or treatment was observed in this assay. All data are shown as mean ± SEM. * p<0.05. CCI + B cells, n = 12 mice; CCI + T cells, *n* = 12 mice; CCI + saline, *n* = 12 mice; sham-treated + B cells, *n* = 10 mice; sham-treated + saline, *n* = 10 mice.

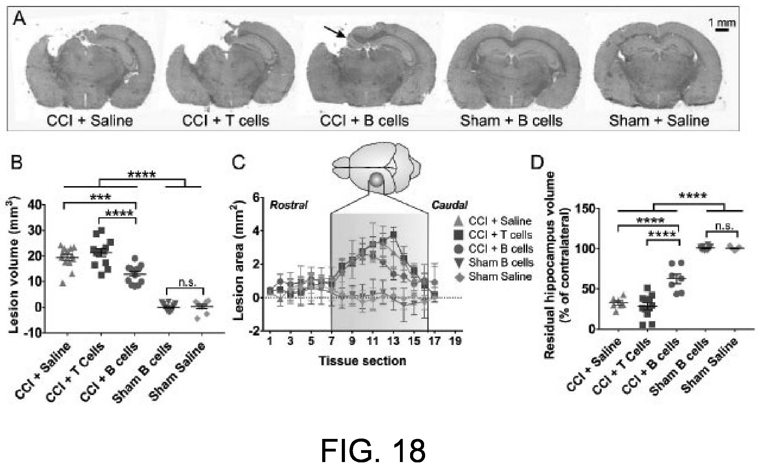

FIG. 18 shows effect of B cell treatment on histological outcome after CCI. (A) Representative coronal sections through the lesion site at day 35 post injury. In B cell-treated animals a portion of the hippocampus was often spared in the lesioned hemisphere (*arrow*)*.* Sections shown are located approximately -2.2 mm from bregma. (B) The total volume of the brain lesion in mice treated with B cells was significantly reduced by 40-60% at 35 days post-TBI as compared to saline and T cell controls. (C) Lesion areas in transverse brain sections along the rostro-caudal axis of the brain. Results illustrate a consistently reduced lesion size in lesioned brains that received B cell treatment. (D) The total volume of the hippocampus spared in the injured hemisphere was significantly higher in B cell-treated animals as compared to either of the CCI controls. All data are shown as mean ± SEM. * p<0.05; ** p<0.01; *** p<0.001; **** p<0.0001. CCI + B cells, *n* = 12 mice; CCI + T cells, *n* = 12 mice; CCI + saline, *n* = 12 mice; sham-treated + B cells, *n* = 10 mice; sham-treated + saline, *n* = 10 mice.

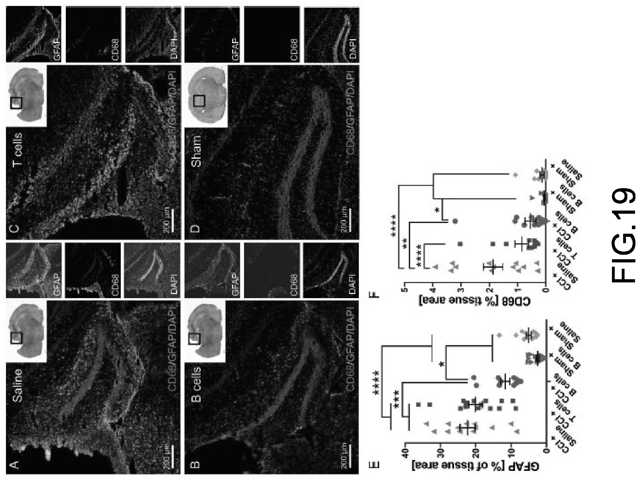

FIG. 19 shows effect of B cell treatment on gliosis and microglial activation. (A-D) Confocal images showing immunolabeling for GFAP and CD68 in overviews of the medial aspect of the injury at 35 days post CCI and treatment with either saline (A), B cells (B), T cells (C), or in saline-treated sham-injured controls (D). (E) Quantitative analysis of the area covered by GFAP immunostaining showed a significant reduction in reactive astrogliosis in injured animals treated with B cells as compared to either saline- or T cell-treated CCI controls. (F) Quantitative analysis of CD68 immunostaining showed a significant reduction in the presence of CD68 in animals treated with either T cells or B cells after CCI as compared to saline-treated CCI controls. *n* = 4 imaging fields per animal. All data are shown as mean ± SEM. * p<0.05; ** p<0.01; *** p<0.001; **** p<0.0001.

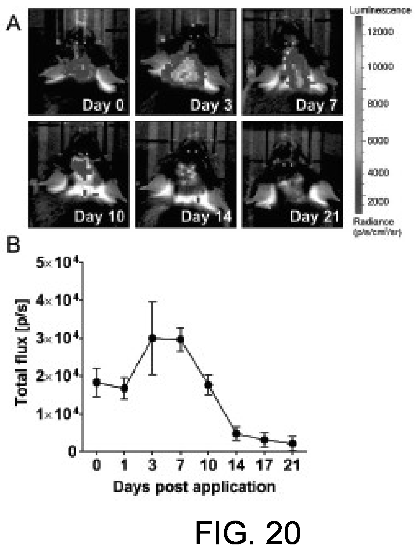

FIG. 20 shows B cell survival and persistence in the brain. (A) Representative example of intravital imaging of a WT C57B16/J mouse at multiple time points after CCI and intraparenchymal application of 5 x 10⁶ B^{luc} cells. (B) Light emission from the head at the site of the CCI injury site (*n* = 6 mice) indicates that the cells survive *in situ* up to approximately 14 days after application, with numbers of viable cells decreasing markedly after day 7. [p/s] = photons per second.

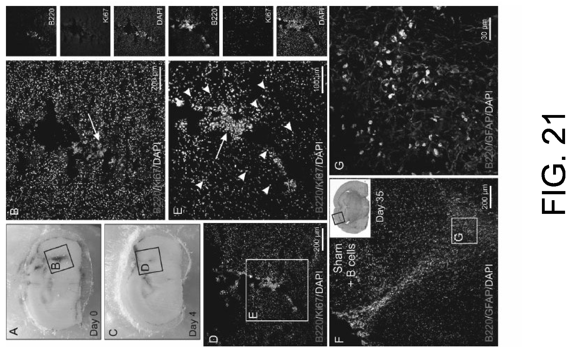

FIG. 21 shows B cell localization at the injury site after CCI. (A) Immediately after intraparenchymal injection and CCI, the pre-labeled B cells can be visualized at the injury site. The black square indicates the area imaged in B. (B) Confocal microscopy image of a coronal section through the lesion site showing B220+ B cells clustered at the injection site (arrow). No cell proliferation, indicated by Ki67 immunolabeling, was observed immediately after injury. (C) Four days after B cell injection and CCI, the labeled B cells can still be observed at the injury site, although the intensity of the vital stain coloration had diminished by this time point compared to immediately after injection. The black square indicates the area imaged in D. (D) Confocal image of a coronal section through the injection site four days post injury and B cell administration. The B220+ B cells can still be found in large numbers clustered at the injury site. Abundant cell proliferation can be observed throughout the region, however no co-staining of B220 and Ki67 was observed. (E) Magnified view of the area boxed in D. (F) In sham-lesioned animals, the needle track through the cortex can still be found 35 days post-treatment outlined by astroglial scarring. No B220+ B cells can be observed at this time point at the original site of injection. (G) High-magnification confocal image of the area boxed in F. In all confocal images, cell nuclei are counterstained with DAPI. *n* = 4 animals per time point.

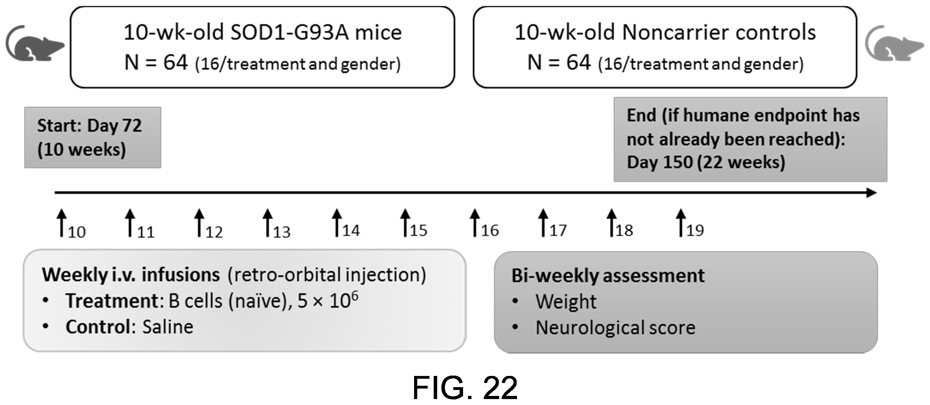

FIG. 22 shows an overview of experimental design. Weight and Neuroscore assessments were performed twice weekly, at the same time of day, by an experimenter who was blinded to the treatment conditions.

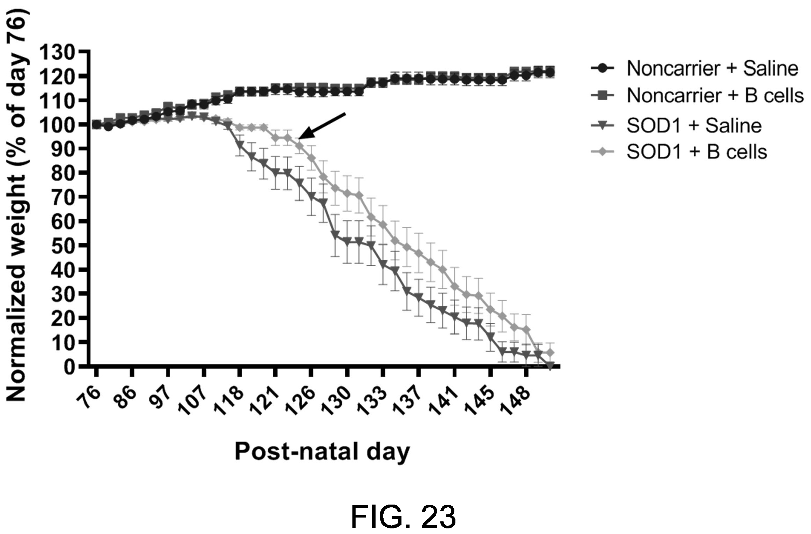

FIG. 23 shows normalized weight (percentage of value at day 76 for each individual animal) over time in the B cell and saline treatment groups, measured twice weekly. The graph shows a composite measure of normalized weight and survival in which individuals that died received weight values of 0. We observed the expected divergence in weight between the noncarrier control animals and the SOD1 transgenic ones, in each of the treatment groups. In non-carrier control animals gradual weight gain was observed over the course of the study, irrespective of treatment. The results also illustrate a delay in the decline for transgenic SOD1 animals that received B cells (arrow). N = 32 per treatment condition.

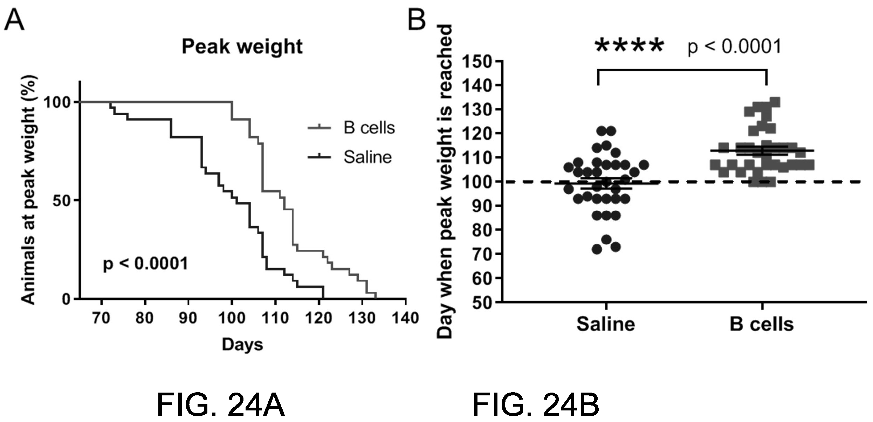

FIG. 24A and FIG. 24B show an analysis of peak weight in SOD1-G93A animals. A. The survival plot indicates the time point at which animals reach their peak body weight. B. Treatment with B cells significantly delayed the onset of paralysis symptoms, indicated by the time at which peak weight is reached. Statistics: A: Gehan-Breslow-Wilcoxon test; B: unpaired t-test. N = 32 animals per group.

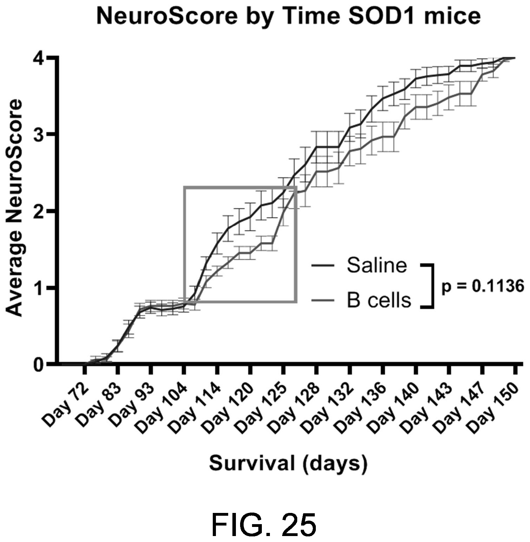

FIG. 25 shows neuroscore values over time. Composite graph of neurological score and survival, in which individuals that died, having reached a Neuroscore of 4, were further assigned values of 4 for the remaining duration of the study. The increase in Neuroscore values typically observed in transgenic SOD1 animals was slowed in animals treated with B cells, particularly in the early stages of disease progression (orange square). Statistics: two-way ANOVA with Tukey post-hoc correction for multiple comparisons.

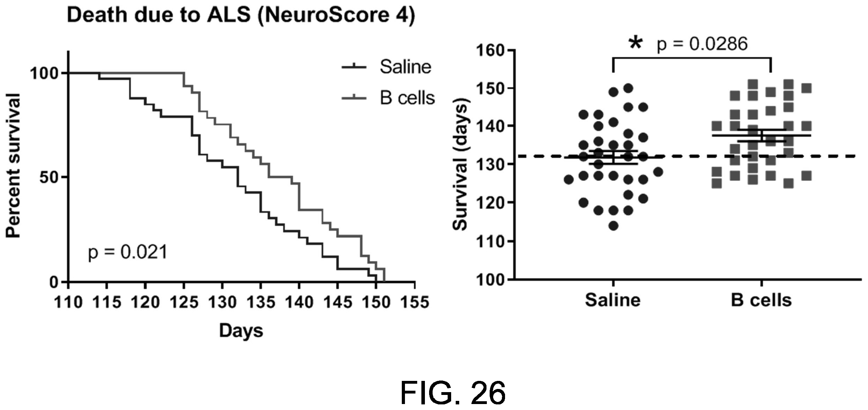

FIG. 26 shows survival analysis of transgenic SOD1-G93A animals. Animals that reached a Neuroscore of 4 (full paralysis) were considered to have died due to ALS. Treatment with naïve B cells significantly extended survival as compared to saline control treatment (N = 32 animals per group). Statistics: (left): Gehan-Breslow-Wilcoxon test; (right): one-way ANOVA. N = 32 animals per group.

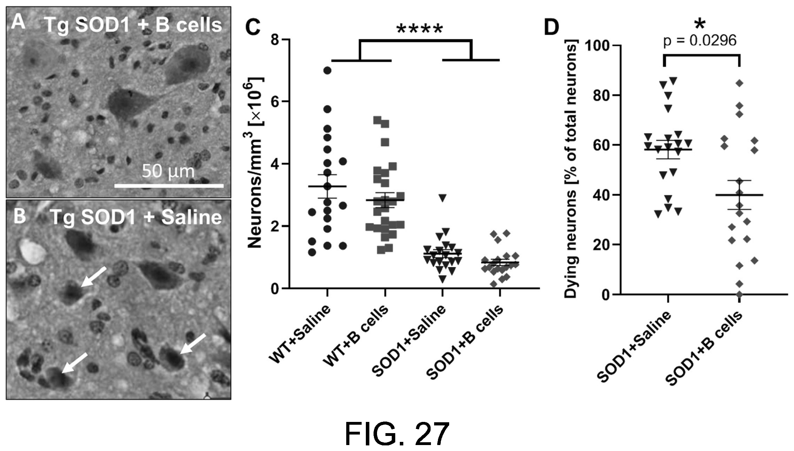

FIG. 27 shows end-point motor neuron evaluation in the lumbar spinal cord. A, B: Lumbar spinal cord sections were stained with H&E and all motor neurons (large cell bodies, at least one nucleolus) as well as impaired, abnormal neurons showing morphological characteristics of injury/degeneration (arrows) were counted by experimenters blinded to the treatments. C: total numbers of motor neurons were significantly reduced in transgenic animals but did not differ with treatment within this group. D: When the percentage of degenerating, pyknotic motor neurons was specifically analyzed, a significant benefit of B cell treatment became apparent. Statistics: (left): two-way ANOVA; (right): unpaired t-test. N = 19-24 animals per group. Note that collection of tissue samples was not possible in all tested animals.

### DETAILED DESCRIPTION

The invention will now be described in detail by way of reference only using the following definitions and examples. Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. Numeric ranges are inclusive of the numbers defining the range.

The headings provided herein are not limitations of the various aspects or embodiments of the invention which can be had by reference to the specification as a whole. Accordingly, the terms defined immediately below are more fully defined by reference to the specification as a whole.

### Definitions

As used herein, the term "neurodegenerative disease" refers to a neurologic disease, disorder, or condition characterized by the progressive loss of structure or function of neurons, including death of neurons, e.g., in the central nervous system (CNS). Many similarities appear that relate these diseases to one another on a sub-cellular level. Moreover, there are many parallels between different neurodegenerative disorders including atypical protein assemblies as well as induced cell death. Neurodegeneration can be found in many different levels of neuronal circuitry ranging from molecular to systemic. Neurodegeneration may be characterized by molecular markers of disease progression, such as, T-tau (total tau), P-tau (hyperphosphorylated tau), Aβ42 (amyloid beta 42), the ratio of Aβ42/Aβ40, YKL-40 (Chitinase-3-like protein 1), VLP-1 (visinin-like protein 1), NFL (neurofilament light), pNFH (phosphorylated neurofilament heavy subunit), Ng (neurogranin) and UCH-L1 (ubiquitin C-terminal hydrolase), TDP-43 (TAR DNA-binding protein 43), decreased α-synuclein and/or decreased levels of 3,4-dihydroxyphenylacetate (see, e.g., Robey and Panegyres. Cerebrospinal fluid biomarkers in neurodegenerative disorders. Future Neurol. 14(1). (2019)). Exemplary neurodegenerative disorders include amyotrophic lateral sclerosis (ALS), Parkinson's disease, Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease.

As used herein, the term "central nervous system (CNS) injury" refers to an injury that disrupts the normal function of the brain and/or the spinal cord. CNS injuries may result from external mechanical force as described herein. CNS injuries include traumatic brain injury (TBI) and/or spinal cord injury (SCI).

As used herein, the term "traumatic brain injury (TBI)" refers to disruption in the normal function of the brain resulting from external mechanical force. For example, TBI may result from a head injury, or a cerebral contusion (e.g., resulting from a fall, a firearm wound, a sports accident, a construction accident, a vehicle accident, or an injury which penetrates the skull or brain of the subject). TBI is diagnosed according to clinical guidelines known to those of skill in the art. TBI may further be characterized by molecular markers of disease progression, such as, protein biomarkers for neuronal cell body injury (UCH-L1, NSE), astroglial injury (GFAP, S100B), neuronal cell death (all-spectrin breakdown products), axonal injury (NF proteins), white matter injury (MBP), post-injury neurodegeneration (total Tau and phospho-Tau), post-injury autoimmune response (brain antigen-targeting autoantibodies) (see, e.g., Wang et al. An update on diagnostic and prognostic biomarkers for traumatic brain injury. Expert Rev Mol Diagn. 18(2): 165-180 (2018)). TBI may be co-incident with SCI and may result from the same injury or accident.

As used herein, the term "spinal cord injury (SCI)" refers to an injury to the spinal cord resulting from external mechanical force. For example, SCI may result from a spinal injury, or a spinal contusion (e.g., resulting from a fall, a firearm wound, a sports accident, a construction accident, a vehicle accident, or an injury which penetrates the spinal cord of the subject). SCI is diagnosed according to clinical guidelines known to those of skill in the art. SCI may be-coincident with TBI and may result from the same injury or accident.

As used herein, the term "inflammatory disease" or "immune disease" refers to a disease, disorder, or condition having an inflammatory or an immune component to the etiology, pathogenesis, progression, or symptomology of the disease. For example, inflammatory or immune disorders may include dysregulation of an inflammatory or immune pathway and/or an abnormal inflammtory or immune response to a stimulus. Exemplary inflammatory or immune disorders include cystic fibrosis, cardiovascular disease, keratoconus, keratoglobus, osteoarthritis, osteoporosis, pulmonary arterial hypertension, retinitis pigmentosa, and rheumatoid arthritis

As used herein, the term "neuroprotective" refers to the property of preventing, inhibiting, or reducing neuronal cell death. For example, a composition or method that is neuroprotective may be characterized by an alteration (e.g., a reduction) in a symptom associated with a neurodegenerative disorder, TBI, or SCI. Alternately, a composition or method that is neuroprotective may be characterized by its effect on a molecular marker of disease, such as those described herein for neurodegenerative disorders, TBI, or SCI.

As used herein, the term "anti-inflammatory" refers to the property of preventing, inhibiting, or reducing inflammation. For example, a composition or method that is anti-inflammatory may be characterized by an alteration (e.g., a reduction) in a symptom associated with an inflammatory disorder. Alternately, a composition or method that is anti-inflammatory may be characterized by a reduction in an inflammatory marker (e.g., a reduction in pro-inflammatory cytokines) or an increase in an anti-inflammatory marker (e.g., an increase in anti-inflammatory cytokines).

As used herein, the term "immunomodulatory" refers to the property of initiating or modifying (e.g., increasing or decreasing) an activity of a cell involved in an immune response. An immunomodulatory composition or method may increase an activity of a cell involved in an immune response, e.g., by increasing pro-inflammatory markers such as cytokines, and/or may decrease an activity of a cell involved in an immune response, e.g., by decreasing pro-inflammatory markers such as cytokines.

As used herein, the term "B cell" or "B lymphocyte," as used interchangeably herein, refers to a type of white blood cell of the small lymphocyte subtype. B cells, unlike the other two classes of lymphocytes, T cells and natural killer cells, express B cell receptors (BCRs) on their cell membrane. BCRs allow the B cell to bind to a specific antigen, against which it will initiate an antibody response. B cells function in the humoral immunity component of the adaptive immune system by secreting antibodies. Additionally, B cells present antigen (they are also classified as professional antigen-presenting cells (APCs)) and secrete cytokines. In mammals, B cells mature in the bone marrow.

As used herein, the term "mature B cells" refers to a B cell that has completed the process of B cell maturation, for example, in the bone marrow of a mammal. Mature B cells leave the bone marrow and migrate to secondary lymphoid tissues, where they may interact with exogenous antigen and or T helper cells. The stages of B cell maturation have been well-characterized in the scientific literature and are known to those of skill in the art.

As used herein, the term "naïve B cell" refers to a B cell that has not been exposed to an antigen.

As used herein, the term "Breg cell" or "B regulatory cell" refers to a type of B cell which participates in immunomodulation and in suppression of immune responses. Breg cells of the disclosure are mature, naïve B cells, expressing characteristic cell surface markers. Breg cells may express one or more of B220, CD1d, CD5, CD19, CD20, CD21, CD22, CD23, CD24, CD25, CD27, CD38, CD44, CD48, CD71, CD73, CD138, CD148, CD274, IgM, IgG, IgA, and IgD. In particular, Breg cells may express cell surface markers including but not limited to B220, CD19, CD20, CD24, IgM, IgD, and CD138. Upon introduction into an injured environment, Breg cells can produce immunomodulatory cytokines including but not limited to IL-2, IL-4, IL-6, IL-10, IL-35, TNF-alpha, TGF-beta, interferon-gamma. In particular, Breg cells are characterized by the production of IL-10.

As used herein, the term "cytokine" refers to refers to a small protein involved in cell signaling. Cytokines can be produced and secreted by immune cells, such as T cells, B cells, macrophages, and mast cells, and include chemokines, interferons, interleukins, lymphokines, and tumor necrosis factors. As used herein, the term "pro-inflammatory cytokine" refers to a cytokine secreted from immune cells that promotes inflammation. Immune cells that produce and secrete pro-inflammatory cytokines include T cells (e.g., Th cells) macrophages, B cells, and mast cells. Pro-inflammatory cytokines include interleukin-1 (IL-1, e.g., IL-1β), IL-5, IL-6, IL-8, IL-10, IL-12, IL-13, IL-18, tumor necrosis factor (TNF, e.g., TNFα), interferon gamma (IFNy), and granulocyte macrophage colony stimulating factor (GMCSF).

As used herein, the term "Toll-like receptor (TLR) agonist" refers to ligands that bind to and activate Toll-like receptors (TLRs), leading to downstream TLR cell signaling. TLR agonists are known to those of skill in the art and include endogenous and exogenous ligands. Exemplary endogenous ligands which are TLR agonists include heat shock proteins, necrotic cells or a fragment thereof, oxygen radicals, urate crystals, mRNA, beta-defensin, fibrin, fibrinogen, Gp96, Hsp22, Hsp60, Hsp70, HMGB1, lung surfactant protein A, low density lipoprotein (LDL), pancreatic elastase, polysaccharide fragment of heparan sulfate, soluble hyaluronan, alpha A-crystallin, and CpG chromatin-lgG complex. Exemplary exogenous ligands which are TLR agonists include Pam3CSK4, triacylated lipopeptide, glycosylphosphatidylinositol (GPI)-anchored protein, lipoarabinomannan, outer surface lipoprotein, lipopolysaccharide, cytomegalovirus envelope protein, glycoinositolphospholipids, glycolipids, GPI anchor, Herpes simplex virus 1 or a fragment thereof, lipoteichoic acid, mannuronic acid polymer, bacterial outer membrane porin, zymosan, double-stranded RNA, single-stranded RNA, Poly(I).Poly(C), taxol, flagellin, modulin, imidazoquinolines, antiviral compounds, unmethylated CpG oligodeoxynucleotide, and profilin.

As used herein, the term "treatment" (and variations thereof, such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of the disease or disorder (such as those described herein), alleviation of symptoms of such diseases, diminishment of any direct or indirect pathological consequences of the diseases, as well as altering an immune response. Additionally, treatment refers to clinical intervention relating to any of the diseases or conditions described herein.

As used herein, by the term "administering" is meant a method of giving a dosage to a subject. The compositions utilized in the methods described herein can be administered, for example, intravitreally (e.g., by intravitreal injection), by eye drop, intramuscularly, intravenously, intradermally, percutaneously, intraarterially, intraperitoneally, intralesionally, intracranially, intraarticularly, intraparenchymally, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, orally, topically, transdermally, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. For topical administration, a dosage is administered in a lotion, a cream, an ointment, or a gel. The method of administration can vary depending on various factors (e.g., the composition being administered, and the severity of the condition, disease, or disorder of immune dysregulation being treated).

A subject to be treated according to this invention is a mammal. The mammal could be, for example, a primate (e.g., a human), a rodent (e.g., a rat or a mouse), or a mammal of another species (e.g., farm or other domesticated animals). In each one of the above methods, the mammal may be one that suffers from any of the diseases or disorders disclosed herein. In preferred embodiments, the subject is a human.

A mammal "in need" of treatment can include, but are not limited to, mammals that have neurodegenerative disorders, TBI, SCI, immunological disorders, mammals that have had immunological disorders, or mammals with symptoms of immunological disorders or mammals having inflammatory disorders or diseases. Exemplary disorders are disclosed herein.

An "effective amount" of an agent, e.g., a pharmaceutical formulation, refers to an amount effective, at dosages and for periods of time necessary, to achieve the desired therapeutic or prophylactic result or a specifically state purpose. An "effective amount" can be determined empirically and by known methods relating to the stated purpose.

The terms "isolating" or "isolation" refer to both the physical identification and the isolation of a cell or cell population from a cell culture or a biological sample. Isolating can be performed by applying appropriate cell biology technologies that are either based on the inspection of cell cultures and on the characterization (and physical separation when possible and desired) of cells corresponding to the criteria, or on the automated sorting of cells according to a characteristic such as the presence/absence of antigens and/or cell size (such as by FACS). In some embodiments, the terms "isolating" or "isolation" may comprise a further step of physical separation and/or quantification of the cells, especially by carrying out flow cytometry. Physical separation also includes enrichment for a particular characteristic of the cell or cell population. An "isolated" cell or population of cells is a cell or population of cells that has been identified and/or separated as described above.

The terms "cell population" or "population of cells" refer generally to a group of cells. Unless indicated otherwise, the term refers to a cell group consisting essentially of or comprising cells as defined herein. A cell population may consist essentially of cells having a common phenotype or may comprise at least a fraction of cells having a common phenotype. Cells are said to have a common phenotype when they are substantially similar or identical in one or more demonstrable characteristics, including but not limited to morphological appearance, the level of expression of particular cellular components or products (e.g., RNA or proteins), activity of certain biochemical pathways, proliferation capacity and/or kinetics, differentiation potential and/or response to differentiation signals or behavior during in vitro cultivation. Such demonstrable characteristics may therefore define a cell population or a fraction thereof. A cell population may be "substantially homogeneous" if a substantial majority of cells have a common phenotype. A "substantially homogeneous" cell population may comprise at least 60%, e.g., at least 70%, at least 80%, at least 90%, at least 95%, or even at least 99% of cells having a common phenotype, such as the phenotype specifically of a B cell (e.g., a B_{reg} cell). Moreover, a cell population may consist essentially of cells having a common phenotype such as the phenotype of B cell (e.g., a B_{reg} cell) if any other cells present in the population do not alter or have a material effect on the overall properties of the cell population and therefore it can be defined as a cell line. Thus an isolated cell population (or, for example, isolated B cells) typically comprises at least 60%, or between 60% and 99%, or between 70% and 90%, of B cells (or subpopulations of B cells such as B_{reg} cells).

### Collection and Isolation of B Cells

Any source of B cells, also known as B lymphocytes, may be used for collection purposes. Such B cells may be derived from the bone marrow, spleen, lymph nodes, blood or other allogeneic tissues that are sources of B cells, as known to one of ordinary skill in the art. Preferred sources of B cells are bone marrow and blood. Preferably, autologous or allogeneic or xenogeneic B cells are collected.

Using sterile techniques, in one embodiment, bone marrow is preferably obtained from the posterior superior ilium. The B cells obtained may be immediately used after isolation and relative purification, may be stored for subsequent use, or may be cultured for a period of time before use. The B cell population in the bone marrow contains pre-pro-B cells, pro-B cells, pre-B cells, immature B cells, and some mature B cells.

In the present application, the term B cell encompasses pre-pro-B cells, pro-B cells, pre-B cells, immature B cells, and mature B cells. From blood or other tissues, B cells can be isolated using standard techniques known to one of ordinary skill in the art.

Methods to obtain B cells or, for example, precursor B cells from heterogeneous cell populations are known. Many of these techniques employ primary antibodies that recognize molecules on the surface of the desired B cells or B cell precursors and use these antibodies to positively select these cells and separate them from unwanted cells. This technique is known as positive selection.

Other techniques commonly employed use primary antibodies that recognize molecules on the surface of the cells to be separated from the desired B cells or B cell precursors. In this manner, molecules on these unwanted cells are bound to these antisera and these cells are removed from the heterogeneous cell population. This technique is known as negative selection.

A combination of positive and negative selection techniques may be employed to obtain relatively pure populations of B cells or precursor B cells. Such populations are referred to as isolated B cells. As is used herein, relatively pure means at least 60% pure, 65% pure, 70% pure, 75% pure, 80% pure, 85% pure, 88% pure, or higher degrees of purity such as at least 90% pure, at least 95% pure, at least 97% pure, or at least 98% to 99% pure.

Numerous techniques are available to one of ordinary skill to separate antibodies bound to cells. Antibodies may be linked to various molecules that provide a label or tag that facilitates separation. In one embodiment, primary antibodies may be linked to magnetic beads that permit separation in a magnetic field. In another embodiment, primary antibodies may be linked to fluorescent molecules that permit separation in a fluorescent activated cell sorter. Fluorescent and magnetic labels are commonly used on primary and/or secondary antibodies to achieve separation. Secondary antibodies which bind to primary antibodies may be labeled with fluorescent molecules that permit separation of cells in a fluorescence activated cell sorter. Alternatively, metallic microbeads may be linked to primary or secondary antibodies. In this manner, magnets may be used to isolate these antibodies and the cells bound to them.

To achieve positive or negative selection, the heterogeneous cell population is incubated with primary antibodies for a time sufficient to achieve binding of the antibodies to the antigen on the cell surface. If the primary antibodies are labeled, separation may occur at this step. If secondary antibodies are employed, then the secondary (anti-primary) antibodies are incubated with the cells bound to the primary antibodies for a time sufficient to achieve binding of the secondary antibodies to the primary antibodies. If the secondary antibody has a fluorescent label, then the cells are sent through a fluorescence activated cell sorter to isolate the labeled antisera bound to the desired cell. If the secondary antibody has a magnetic label, then the selected cell with the primary antibody and secondary antibody-labeled microbeads form a complex that when passed through a magnet remain behind while the other unlabeled cells are removed along with the cell medium. The positively labeled cells are then eluted and are ready for further processing. Negative selection is the collection of the unlabeled cells that have passed through the magnetic field.

Miltenyi Biotec has developed numerous products for the straightforward magnetic separation of B cells and distinct B cell subsets. B cells can be isolated either straight from whole blood or buffy coat without density gradient centrifugation or erythrocyte lysis, or from peripheral blood mononuclear cells (PBMCs) after density gradient centrifugation. Both positive selection and depletion strategies can be pursued for direct isolation and isolation of B cells according to standard methods.

Thus, in a working embodiment, a patient and a potential donor are HLA (A, B, and DR-B1) tested, for example, by the American Red Cross. Potential donors found to be a haploidentical match to the recipient are taken as useful allogeneic donors. Donors are then subjected to apheresis for separating and collecting B cells. B cell product for infusion is then prepared.

Upon receipt of donor allogeneic mononuclear cells - MNC (A), the apheresis product is enriched for B cells using Miltenyi Biotec CliniMACS^{®} CD19 selection. After platelet wash, the product (up to 4 x 10¹⁰ total cells and up to 5x10⁹ CD19+ cells per vial of CD19 CliniMACS reagent) are processed for CD19+ cells enrichment using CD19 microbeads separated on LS column. The target fraction is washed and the infusion media is then Plasma-Lyte A supplemented with 25% HSA (1% Final Concentration).

The methods, in general, include:

### DAY 1

a. Donor Apheresis Product received and sampled for Sterility, Cell Count, Viability and Flow Cytometry.
b. Product stored refrigerated overnight.

### DAY 2

a. Product removed from refrigerator, mixed well, and left to equilibrate to ambient temperature for 30 minutes. Samples removed for sterility, cell count, viability and Flow Cytometry (DuraClone panel with CD20).
b. Platelet wash performed as per a standard CliniMacs procedure.
c. Beads added and incubated for 30 minutes on a rocker as per standard CliniMacs procedure except that incubation performed at 4°C.
d. Post Bead incubation, 1 Antibody wash performed using chilled (4°C) medium, and product loaded onto the CliniMacs LS column as per standard CliniMacs procedure:
e. Separation run using CliniMacs Enrichment 1.1 program.
f. CD19 Enriched Target fraction sampled for cell count, flow cytometry, stability and sterility
g. CD19 depleted (Non-Target fraction) sampled for cell count and flow cytometry

Isolation of B cells from heterogeneous cell populations and stem cell populations may also involve a negative selection process in which the marrow first undergoes red cell lysis by placing the bone marrow in a hypotonic buffer and centrifuging the red blood cells out of the buffer. The red blood cell debris remains in the supernatant which is removed from the test-tube. The bone marrow derived cells are then resuspended in a buffer that has the appropriate conditions for binding antibody. Alternatively, the bone marrow can be subjected to a density gradient centrifugation. The buffy coat layer containing the bone marrow derived cells is removed from the gradient following the centrifugation. The cells are washed and resuspended in the antibody binding buffer and is then incubated with primary antibodies directed toward stem cells, T cells, granulocytes and monocytes/macrophages (called lineage depletion) followed by positive selection using antibodies toward B cells.

Different B-cell subpopulations can be distinguished on the basis of differential expression of various surface markers and collected accordingly.

### Ex vivo stimulation of B Cells

Once isolated, B cells may be treated or stimulated by exposing them to one or more TLR agonists or immunomodulatory cytokine as is described herein. Production of IL-10 producing B_{reg} cells using such ex vivo stimulation is taken useful in the methods and therapeutic strategies described herein.

### Administration

The number of cells to be administered will be related to the area or volume of affected area to be treated, and the method of delivery.

One non-limiting range of B cell number for administration is 10⁴ to 10¹⁴ B cells, depending on the volume of tissue or organ to be treated. Other ranges include 10⁵ to 10¹² B cells and 10⁸ to 10¹⁰ B cells. A pharmaceutical composition including B cells (e.g., B_{reg} cells) may include 10⁴ to 10¹⁴ B cells, 10⁵ to 10¹² B cells, or 10⁶ to 10¹⁰ B cells in a single dose.

Individual injection volumes can include a non-limiting range of from 1 µl to 1000 µl, 1 µl to 500 µl, 10 µl to 250 µl, or 20 µl to 150 µl. Total injection volumes per animal range from 10 µl to 10 ml depending on the species, the method of delivery and the volume of the tissue or organ to be treated.

### Pharmaceutical Compositions

The B cells described herein may be incorporated into a vehicle for administration into a patient, such as a human patient suffering from a disease or condition described herein. Pharmaceutical compositions containing B cells can be prepared using methods known in the art. For example, such compositions can be prepared using, e.g., physiologically acceptable carriers, excipients or stabilizers (Remington: The Science and Practice of Pharmacology 22nd edition, Allen, L. Ed. (2013)), and in a desired form, e.g., in the form of aqueous solutions.

The B cells described herein can be administered in any physiologically compatible carrier, such as a buffered saline solution or a solution containing one or more electrolytes (e.g., one or more of sodium chloride, magnesium chloride, potassium chloride, sodium gluconate, or sodium acetate trihydrate). For example, the B cells may be administered in a PlasmaLyte infusion buffer. PlasmaLyte is a family of balanced crystalloid solutions with multiple different formulations available worldwide according to regional clinical practices and preferences. It closely mimics human plasma in its content of electrolytes, osmolality, and pH. PlasmaLyte solutions also have additional buffer capacity and contain anions such as acetate, gluconate, and even lactate that are converted to bicarbonate, CO₂, and water. The advantages of PlasmaLyte include volume and electrolyte deficit correction while addressing acidosis. In preferred embodiments, the infusion buffer is PlasmaLyte A. PlasmaLyte A is a sterile, nonpyrogenic isotonic solution for injections (e.g., intravenous) administration. Each 100 mL of PlasmaLyte A contains 526 mg of Sodium Chloride (NaCl); 502 mg of Sodium Gluconate (C₆H₁₁NaO₇); 368 mg of Sodium Acetate Trihydrate, (C₂H₃NaO₂•3H₂O); 37 mg of Potassium Chloride (KCl); and 30 mg of Magnesium Chloride (MgCl₂•6H₂O). It contains no antimicrobial agents. The pH is adjusted with sodium hydroxide. The pH is about 7.4 (e.g., 6.5 to 8.0).

Other pharmaceutically acceptable carriers and diluents include saline, aqueous buffer solutions, solvents and/or dispersion media. The use of such carriers and diluents is well known in the art. Other examples include liquid media, for example, Dulbeccos modified eagle's medium (DMEM), sterile saline, sterile phosphate buffered saline, Leibovitz's medium (L15, Invitrogen, Carlsbad, Calif.), dextrose in sterile water, and any other physiologically acceptable liquid.

Dispersions may also be prepared in glycerol, liquid polyethylene glycols, and mixtures thereof and in oils. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. In many cases, it will be preferable to include isotonic agents, for example, sugars or sodium chloride. The solution is preferably sterile and fluid to the extent that easy syringability exists. Preferably, the solution is stable under the conditions of manufacture and storage and preserved against the contaminating action of microorganisms such as bacteria and fungi through the use of, for example, parabens, chlorobutanol, phenol, ascorbic acid, thimerosol, and the like. Solutions of the invention can be prepared by using a pharmaceutically acceptable carrier or diluent and, as required, other ingredients enumerated above, followed by filtered sterilization, and then incorporating the B cells as described herein.

For example, a solution containing a pharmaceutical composition described herein may be suitably buffered, if necessary, and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. The person responsible for administration will, in any event, determine the appropriate dose for the individual subject. Moreover, for human administration, preparations may meet sterility, pyrogenicity, general safety, and purity standards as required by FDA Office of Biologics standards.

The pharmaceutical composition may also include an excipient to promote cell membrane stability. The infusion medium may be supplemented with, for example, a highly soluble osmolytic protein, such as a highly soluble osmolytic protein with a high molecular weight. Serum proteins, such as Human Serum Albumin (HSA), may be included in a pharmaceutical composition described herein as a medium supplement for maintaining cell membrane stability. HSA includes recombinant albumin. Alternately, human serum may be used to stabilize pharmaceutical compositions including cells.

### EXAMPLES

The present invention is described in further detail in the following examples which are not in any way intended to limit the scope of the invention as claimed. The attached Figures are meant to be considered as integral parts of the specification and description of the invention. The following examples are offered to illustrate, but not to limit the claimed invention.

### Example 1: Exogenous B Cells Modulate Immune Infiltration and Response

This example, using isobaric-labeling multiplexed proteomics, illustrates a large-scale analysis of the molecular impact of B cells in wound healing.

Our data showed that B cell application has a significant homeostatic effect on the wound microenvironment, with a strong reduction in proteins associated with inflammatory response, as well as an increase in proteins associated with tissue growth and remodeling. By retrieving the applied exogenous B cells from the wound niche at various time points after application and examining the cell populations through multi-color flow cytometry, we determined that mature naïve B cells applied into a wound shift towards a regulatory phenotype characterized by expression of CD138 and the immune-regulatory cytokines IL-10, IL-35, and TGF-β. This Breg-like phenotype appeared transient, with a peak at 2 days after application. Moreover, the phenotype of monocytes and macrophages in the wound environment was significantly altered as a result of B cell application, with reduced expression of pro-inflammatory cytokines including IL-2, IL-4, IL-6, and IFN-γ. Accordingly naïve B cells placed at the site of an injury detect local inflammatory signals and damage-associated molecular patterns (DAMPs) via TLR- and B cell receptor (BCR)-dependent pathways, and adopt a regulatory phenotype associated with production of anti-inflammatory cytokines, preferably IL-10, but also IL-4, IL-35, and TGF-β, that act on adjacent immune cells and fibroblasts and bias their phenotype towards an anti-inflammatory, pro-regenerative one. Indeed, wound healing studies showed that B cells deficient in the common TLR-signaling adaptor myeloid differentiation factor 88 (MyD88) or deficient in IL-10 lose their pro-regenerative capacity.

### MATERIALS AND METHODS

The following materials and methods were utilized for this study.

### Animals

Wound healing studies were conducted in 7-9 weeks-old male wild-type C57B16/J mice (Jackson Laboratories). Male WT C57BI6/J were used as isogeneic donors for B cell isolation. Animals were maintained under standard laboratory care conditions, at temperatures ranging between 20-23°C, under a 12h:12h light:dark cycle, with ad libitum access to food and acidified water. All animal procedures were performed following the Public Health Service Policy on Humane Care of Laboratory Animals and approved by the Institutional Animal Care and Use Committee of Massachusetts General Hospital. All efforts were made to reduce the number of animals used and to minimize animal suffering.

### Cell isolation

Mouse spleens were collected in ice cold EasySep^{™} buffer (STEMCELL Technologies) containing 2% fetal bovine serum (FBS) and 1 mM ethylenediaminetetraacetic acid (EDTA) in phosphate-buffered saline (PBS). Spleens were dissociated mechanically through a 40 µm cell strainer and the splenocyte suspension was processed for negative B or T cell selection through immunomagnetic separation, using commercially available cell isolation kits (STEMCELL Technologies) according to the manufacturer's instructions.

### Wound model and tissue sampling

Full-thickness excisional wounds through the dorsal skin were induced as described previously (Wang et al. (2013) Nat Protoc. 8(2):302-9.). Briefly, mice were anesthetized with a cocktail of ketamine (100 mg/kg) and xylazine (10 mg/kg), and the dorsal skin was shaved and depilated. Analgesia was provided pre-operatively with 0.08 mg/kg buprenorphine injected subcutaneously. The dorsal skin was tented and a 5-mm biopsy punch was passed through the skin fold, creating two symmetrical wounds on each side of the back. Each wound had an initial area of approximately 20 mm². Silicone splints with an inner diameter of 7 mm (Sigma-Aldrich) were attached around the wounds using Vetbond tissue adhesive (3M). The splinted wounds were then covered with Tegaderm^{™} transparent dressing (3M). Cell suspensions in PBS or equal volumes of PBS solution alone (saline control) were applied directly onto the wound bed using a manual pipette. Each mouse also received two localized subcutaneous injections under the dorsal skin with equal doses of B cells or saline solution. Each treated wound or subcutaneous site received 15-20 × 10⁶ B cells in 20 µl PBS.

After defined time intervals, 18 hours, 2 days, or 4 days, the mice were lightly anesthetized using 3% isoflurane in O₂, and 10-20 µl of a working solution of brefeldin A (GolgiPlugTM, BD Pharmingen) in PBS was applied to each of the treated wound and subcutaneous sites in order to facilitate the accumulation of cytokines within cells. After 4 hours of incubation, the mice were euthanized and tissue biopsies including the wounds and the subcutaneous injection sites were collected. Tissue biopsies were enzymatically dissociated for 30 minutes at 37°C with gentle rocking in RPMI medium containing 5% FBS, 0.5% L-glutamine, 0.5% penicillin-streptomycin, 1.5 mg/ml, 0.25 U/mg Collagenase D (Roche), 1.5 mg/ml, >400 U/mg hyaluronidase from bovine testes (Millipore Sigma), 0.4 mg/ml, 400 U/mg DNAse I (Roche), 0.025 mg/ml, >10 U/mg Dispase I (Millipore Sigma). Tissues were then mechanically minced into smaller pieces, followed by further enzymatic dissociation for another 30 minutes in the same solution at 37°C with gentle rocking. The digested tissues from individual wound and subcutaneous samples were then pooled for each mouse, and passed through a 100 µm cell strainer followed by a 40 µm cell strainer, resulting in single-cell suspensions.

### Proteomics

**Tissue sampling.** Full-thickness excision wounds were generated in the dorsal skin of mice as described above. The wounds were treated either with a solution of purified B cells at 2 × 10⁶ cells suspended in 20 µl saline, or with saline control. After defined time intervals, 0 days (approximately 10 minutes), 1 day, 4 days, and 10 days, the mice (n = 3-5 per condition) were euthanized, and the wound area, including wound margin and subcutaneous layers was excised and flash-frozen in liquid nitrogen, then stored at -80°C until lysis.

**Protein digestion and tandem mass tag (TMT) labeling.** Sample processing was performed as previously described (Lapek et al. (2017) Nat Biotechnol. 35(10):983-989). Protein concentration of the cell lysates was determined with a BCA assay (Thermo Scientific). Proteins were then reduced with DTT and alkylated with iodoacetamide as previously described. Reduced and alkylated proteins were precipitated via methanol-chloroform precipitation. The precipitated proteins were reconstituted in 300 µL of 1 M urea in 50 mM HEPES, pH 8.5. Vortexing, sonication and manual grinding were used to aid solubility. The solubilized protein was digested in a two-step process starting with overnight digestion at room temperature with 3 µg of Lys-C (Wako) followed by 6 h of digestion with 3 µg of trypsin (sequencing grade, Promega) at 37°C. The digest was acidified with trifluoroacetic acid (TFA). The digested peptides were desalted with C18 solid-phase extraction (SPE) (Sep-Pak, Waters). The concentration of the desalted peptide solutions was measured with a BCA assay, and peptides were dried under vacuum in 50-µg aliquots, which were stored at -80 °C until they were labeled with the TMT reagents. TMT reagents (Thermo Scientific) were suspended in dry acetonitrile (ACN) at a concentration of 20 µg/µL. Dried peptides (50 µg) were resuspended in 30% ACN in 200 mM HEPES, pH 8.5, and 5 µL of the appropriate TMT reagent was added to the sample. Peptides were incubated with the reagents for 1 h at room temperature. The labeling reaction was quenched by adding 6 µL of 5% hydroxylamine. Labeled samples were then acidified by adding 50 µL of 1% TFA, and the peptide mixtures were pooled into ten-plex TMT samples. The pooled samples were desalted via C18 SPE on Sep-Pak cartridges as described above.

**Basic pH reversed-phase liquid chromatography (bRPLC) sample fractionation.** Sample fractionation was performed by bRPLC39, and fractions were pooled for analysis by mass spectrometry. Briefly, the samples were resuspended in a solution with 5% formic acid and 5% ACN and separated over a 4.6-mm × 250-mm ZORBAX Extend C18 column (5 µm, 80 Å, Agilent Technologies) on an Agilent 1260 HPLC system outfitted with a fraction collector, degasser and variable- wavelength detector. The separation was performed by applying a gradient build from 22 to 35% ACN in 10 mM ammonium bicarbonate in 60 min at a flow rate of 0.5 mL/min. A total of 96 fractions were combined as previously described (Edwards et al. (2016) Methods Mol Biol. 1394:1-13). The combined fractions were dried under vacuum, reconstituted with a solution of 5% formic acid and 5% ACN, and then analyzed by LC-MS2/MS3 for identification and quantification.

**Liquid chromatography coupled to mass spectrometry.** All LC-MS2/MS3 experiments were conducted on an Orbitrap Fusion (Thermo Fisher Scientific) coupled to an Easy-nLC 1000 (Thermo Fisher Scientific) with a chilled autosampler. Peptides were separated on an in-house-pulled, in-house-packed microcapillary column (inner diameter, 100 µm; outer diameter, 360 µm). Columns were packed first with ~0.5 cm of Magic C4 resin (5 µm, 100 Å, Michrom Bioresources) followed by ~0.5 cm of Maccel C18 AQ resin (3 µm, 200 Å; Nest Group) and then to a final length of 30 cm with GP-C18 (1.8 µm, 120 Å; Sepax Technologies). Peptides were eluted with a linear gradient from 11 to 30% ACN in 0.125% formic acid over 165 min at a flow rate of 300 nL/min while the column was heated to 60 °C. Electrospray ionization was achieved by applying 1,800 V through a PEEK T-junction at the inlet of the microcapillary column.

The Orbitrap Fusion was operated in data-dependent mode, with a survey scan performed over an m/z range of 500- 1,200 at a resolution of 6 × 10⁴ in the Orbitrap. For the MS1 survey scan, automatic gain control (AGC) was set to 5 × 10⁵, the maximum injection time to 100 ms, and the radio frequency (RF) setting of the S-lens was 60. The most abundant ions detected in the survey scan were subjected to MS2 and MS3 experiments using the 'top speed' setting that enables a maximum number of spectra to be acquired in a 5-s experimental cycle before the next cycle is initiated with another survey full-MS scan. For MS2 analysis, the decision-tree option was enabled, with precursors selected based on charge state and m/z range. Doubly charged ions were selected from an m/z range of 600-1,200, as triply and quadruply charged ions had to be detected in an m/z range of 500-1,200. The ion intensity threshold was set to 5 × 10⁵. When acquiring MS2 spectra, ions were isolated by applying a 0.5-m/z window using the quadrupole and fragmented using collision-induced dissociation (CID) at a normalized collision energy of 30%. Fragment ions were detected in the ion trap at a rapid scan rate. The AGC target was set to 1 × 10⁴, and the maximum ion injection time was 35 ms.

MS3 analysis was performed using synchronous precursor selection (MultiNotch MS3) that was enabled to maximize sensitivity for quantification of TMT reporter ions. Up to ten MS2 precursors were simultaneously isolated and fragmented for MS3 analysis. The isolation window was set to 2.5 m/z, and fragmentation was carried out by HCD at a normalized collision energy of 50%. Fragment ions in the MS3 spectra were detected in the Orbitrap at a resolution of 60,000 at m/z ≥110. The AGC target was set to 5 × 10⁴ ions and the maximum ion injection time to 250 ms. Fragment ions in the MS2 spectra with an m/z of 40 m/z below and 15 m/z above the precursor m/z were excluded from being selected for MS3 analysis.

**Data processing and analysis.** Data were processed using an in-house-developed software suite (Huttlin et al. (2010) Cell. 143(7):1174-89). RAW files were converted into the mzXML format using a modified version of ReAdW.exe (http://www.ionsource.com/functional_reviews/readw/t2x_update_readw.htm). Spectral assignments of MS2 data were made using the Sequest algorithm to search the Uniprot database of mouse protein sequences, including known contaminants such as trypsin.

The database was appended to include a decoy database consisting of all protein sequences in reverse order. Searches were performed with a 50-p.p.m. precursor mass tolerance. Static modifications included ten- plex TMT tags on lysine residues and peptide N-termini (+229.162932 Da), and carbamidomethylation of cysteines (+57.02146 Da). Oxidation of methionine (+15.99492 Da) was included as a variable modification. Data were filtered to a peptide and protein false discovery rate (FDR) of <1% using the target-decoy search strategy (Elias et al. (2010) Methods Mol Biol 604: 55-71). This was achieved by first applying a linear discriminator analysis to filter peptide annotations (peptide-spectral matches) using a combined score from the following peptide and spectral properties: XCorr, ΔCn, missed tryptic cleavages, peptide mass accuracy and peptide length. The probability of a peptide-spectral match to be correct was calculated using a posterior- error histogram, the probabilities of all peptides assigned to one specific protein were combined through multiplication, and the data set was re-filtered to a protein assignment FDR of <1% for the entire data set of all proteins identified across all of the samples analyzed. Peptides that matched to more than one protein were assigned to the protein containing the largest number of matched redundant peptide sequences following the law of parsimony.

For quantitative analysis, TMT reporter ion intensities were extracted from the MS3 spectra by selecting the most intense ion within a 0.003-m/z window centered at the predicted m/z value for each reporter ion, and signal-to-noise (S/N) values were extracted from the RAW files. Spectra were used for quantification if the sum of the S/N values of all of the reporter ions was ≥386 and the isolation specificity for the precursor ion was ≥0.75. Protein intensities were calculated by summing the TMT reporter ions for all of the peptides assigned to a protein.

### Flow cytometry

To evaluate cell viability after retrieval from tissue digestion, the cell suspensions were washed and resuspended in PBS and stained using a Zombie UV fixable viability kit (Biolegend, Inc.) for 30 minutes in the dark at 4°C with gentle rocking. The stained cells were then washed and resuspended in PBS containing 1% FBS, 0.01% sodium azide (RICCA Chemical, Arlington, TX) and 5% FcR blocking reagent (Miltenyi Biotec, Inc) for 10 minutes in the dark at 4°C. Blocked cells were then incubated for 30 minutes in the dark at 4°C with the following fluorophore-conjugated primary surface antibodies: Brilliant Violet 785-conjugated rat anti-mouse CD19 (clone 6D5), Alexa Fluor^{®} 700-conjugated rat anti-mouse/human CD45R/B220 (clone RA3-6B2), APC/Cy7-conjugated rat anti-mouse CD138 (clone 281-2) (all from Biolegend, Inc.), Brilliant Ultraviolet 395-conjugated hamster anti-mouse CD69 (clone H1.2F3), PE-CF594-conjugated rat anti-mouse CD140a (clone APA5) (both from BD Biosciences, San Jose, CA). Surface stained cells were washed and resuspended in fixation buffer (Biolegend, Inc.) for 30 minutes at 4°C, followed by permeabilization wash buffer (1X) (Biolegend, Inc.). Permeabilized cells were then incubated for 30 minutes in the dark at 4°C with the following fluorophore-conjugated primary intracellular antibodies: Brilliant Violet 421-conjugated mouse anti-mouse TGF-β1 (clone TW7-16B4), Brilliant Violet 510-conjugated rat anti- mouse IFN-γ (clone XMG1.2), Brilliant Violet 605-conjugated rat anti-mouse IL-4 (clone 11B11), Brilliant Violet 711- conjugated rat anti-mouse TNF-α (clone MP6-XT22), PerCP/Cy5.5-conjugated rat anti-mouse IL-2 (clone JES6-5H4), PE/Cy7-conjugated rat anti-mouse IL-10 (clone JES5-16E3), APC-conjugated rat anti-mouse IL-6 (clone MP5-20F3) (all from Biolegend, Inc.), fluorescein-conjugated rat anti-mouse IFN-β (clone RMMB-1), PE-conjugated rat anti-mouse IL-27/IL-35 EBI3 Subunit (clone 355022) (both from R&D Systems, Minneapolis, MN). Cells were analyzed on an LSRFortessa X-20 flow cytometer (BD Biosciences, San Jose, CA) equipped with BD FACSDIVA^{™} software and 355 nm, 405 nm, 488 nm, 561 nm and 640 nm lasers. At least 100,000 events were collected from each sample for analysis. Data were analyzed using FlowJo software, version 10.3 (TreeStar, Inc., Ashland, OR).

### Immunohistochemistry

Wound biopsies collected at 0 days (intact), 1 day, 4 days, 10 days, and 16 days post-injury were fixed in 4% buffered paraformaldehyde for 24-48 hours at 4°C, then cryoprotected in 1M sucrose solution for another 24-48 hours at 4°C, and embedded in tissue freezing medium (Electron Microscopy Sciences). Transverse sections through the wound bed were cut at a thickness of 10 µm using a cryostat (Leica Biosystems), and thaw-mounted onto SuperFrost Plus Gold slides (Fisher Scientific). For immunohistochemical detection of antigens, sections were washed through three changes of Tris-buffered saline (TBS) pH 7.4, then permeabilized and blocked by incubation for 1 hour at room temperature with TBS containing 5% bovine serum albumin, 5 % FBS, and 0.3 % Triton X-100. Sections were then incubated overnight at 4°C with the following primary antibodies diluted in blocking solution: APC-conjugated rat anti-mouse CD45R/B220 (clone RA3-6B2; BioLegend,lnc.), PE-conjugated rat anti-mouse CD31 (clone MEC 13.3; BD Biosciences), Alexa Fluor^{®} 488-conjugated mouse anti- tubulin β3 (clone TUJ1; BioLegend, Inc.), Alexa Fluor^{®} 488-conjugated rat anti-mouse F4/80 (clone BM8; BioLegend, Inc.), PE-conjugated rat anti-mouse CD11b (clone M1/70; BioLegend, Inc.), rabbit polyclonal anti-Ki67 (Abcam), and rabbit monoclonal anti-activated caspase 3 (clone C92-605; BD Pharmigen). Unbound primary antibody was removed by 3 rinses for 5 min each in TBS. If unconjugated primary antibodies were used, antigenic sites were visualized by incubating the sections for 2 hours at room temperature with Alexa Fluor 488^{®}-conjugated F(ab')2- goat anti-rabbit IgG (Thermo Fisher Scientific), diluted 1:200 in blocking solution. Sections were counterstained by incubation with 2 µg/ml of 4', 6-diamidino- 2-phenylindoledihydrochloride (DAPI; Sigma Aldrich) in PBS for 3 min at room temperature. The sections were washed 3 times for 7 min in TBS and embedded using Fluoromount (Novus Biologicals). Antibody controls included incubation of the tissue sections with isotype antibodies and omission of the primary antibody when a secondary antibody was used for visualization. No unspecific signal was detected in the control samples.
Stained tissue sections were imaged using a Zeiss LSM 710 laser scanning microscope (Carl Zeiss) equipped with 20×, 40× and 63× objectives. Confocal images were taken at a resolution of 0.1-0.7 µm/pixel and an optical thickness of 0.5-2.2 µm using Zen software (Carl Zeiss).

### Histology

Wound biopsies at the end point of the wound healing time course were collected using a 10-mm biopsy punch and fixed in 4% paraformaldehyde in PBS for 24-48 hours at 4°C, after which the samples were dehydrated through graded ethanol and xylene washes and embedded in paraffin. Transverse sections through the wound bed were cut at a thickness of 5 µm and mounted onto microscopy slides. Serial sections were stained with hematoxylin and eosin and with Masson's trichrome stain for visualizing collagen fibers. Stained slides were digitized at a resolution of 0.25 µm/pixel using an Aperio CS2 scanner (Leica Biosystems). Digitized slides were used for scoring of tissue regeneration by an experimenter blinded to the treatment conditions.

### Statistics

The time-dependent effects of B cell application on the expression of cell markers of interest were assessed using linear mixed effects modeling in SPSS 23 (IBM Corporation), separately for each examined cell population. For variance stabilization, the proportions of gated cells for each sample and examined marker were logit- (logarithm of odds) transformed prior to analysis. Survival time (18, 45, or 93 hours), environment (wound, subcutaneous, or, for B cells only, ice), marker, and, if applicable, condition (B-cell treated or saline) were included as fixed factors, using a three-way (or four-way, where applicable) full factorial design. Technical (run date) and biological replicates (mouse / sample ID) were included as random effects. Post-hoc contrasts between fixed-factor levels were adjusted for multiple comparisons using the Dunn-Sidak method. * p < 0.05; ** p < 0.01; *** p < 0.001; **** p < 0.0001.

### RESULTS

### B cell application induces complex changes in the molecular microenvironment of a wound

Proteomic analysis of whole wound lysates identified up to 9125 proteins across all samples and treatments. For analysis between treatment conditions and across time points, only proteins that were present in all samples (n = 30 animals) were considered. This resulted in a total of 3809 proteins (FIG. 12).

A schematic representation of the average duration of the major stages of wound healing in the wild-type murine wound model is shown in FIG. 1A. Heatmaps summarizing the expression dynamics over time in proteins with significantly altered expression in response to B cell application are shown in FIG. 1B. A total of 213 proteins representing the aggregate of significantly altered proteins associated with B cell treatment (n = 111; p < 0.05, unpaired t-test) as well as those proteins with high fold change (top 20 up- or down-regulated proteins) for each time point, regardless of significance level (n = 112), were classified according to processes relevant to wound healing. Heatmaps show fold change expression after B cell treatment at 0, 1, 4, 10 d post-injury. Red = up-regulation; Green = down-regulation. Particularly notable were the down-regulation of multiple proteins associated with inflammation and inflammatory cells at 4 days post-injury and the substantial up-regulation of proteins associated with cell proliferation, protection from apoptosis (cell death) and oxidative stress, and tissue remodeling (formation of hair follicles and muscle) at 4-10 days post injury.

### Expression of proteins by functional family

Average expression of proteins by functional family over time in wounds treated with saline (controls, regular wound healing) or after B cell treatment are shown in Figs. 2A through 2H. This analysis illustrates the overall effect of B cells as a homeostatic agent, rather than an inducer or inhibitor of protein expression. B cell application was associated with the maintenance of steady levels of expression of proteins that normally either decline or increase during the course of injury and healing, significantly reducing the inflammatory peak observed in normal healing, preventing the reduction in anti-apoptotic factors (arrows) and oxidative stress protectants and increasing proliferation (FIG.2A - FIG.2B), reducing the decline in anti-oxidative stress protectants and in cell proliferation, and maintaining cell migration at lower levels (FIG. 2C - FIG. 2D), maintaining steady levels of proteins associated with remodeling and secondary skin structures (FIG. 2E - FIG. 2F), reducing levels of protein degradation and autophagy observed in the early stages of injury in controls, and increasing levels of proteins associated with angiogenesis and nerve regeneration at late stages of healing (FIG. 2G - FIG. 2H).

An unsupervised hierarchical cluster analysis of identified proteins expressed in skin wound samples is depicted in FIG. 12. Only identified proteins that were found consistently present across all samples were included in the analysis. Hierarchical clustering using complete linkage of 3809 proteins (rows) consistently expressed in all animals in both B cell and saline treated wounds at 4 different time points after injury (columns) is shown in FIG. 12A. The pseudocolor scale depicts normalized, log-transformed fold change expression values for each protein. The dendrogram shows 15 protein clusters derived from this analysis, with the color of each cell in (FIG. 12A) mapped to the mean expression value of the cluster at the respective time points. Proteins cluster by their pattern of expression over time. In FIG. 12B a heatmap of hierarchical cluster from (FIG. 12A) showing all 3809 proteins is depicted. Gene ontology analysis of the 15 hierarchical clusters is shown in FIG. 12C. The mouse GOslim gene list from QuickGO (accessible at https://www.ebi.ac.uk/QuickGO) was used to probe the 15 hierarchical clusters. Bar graphs show the top biological function categories for each cluster.

Distribution of significantly altered proteins in response to B cell treatment at each assessed time point during wound healing was determined and is shown in Figure 13.

### In vivo assessment of B cell application in acute wound healing

An experimental paradigm for in vivo assessment of B cell application in acute wound healing is depicted in FIG. 3. A total of 4 full-thickness lesions were generated in the dorsal skin of a wild-type C57BI6 mouse and mature naïve B cells purified from isogeneic animals were applied directly on the wound beds. Control animals received saline applications. As internal control, B cells or saline control were also injected subcutaneously under intact skin to provide a similar microenvironment without the injury. After defined survival times, the wound or cutaneous uninjured tissue was collected, dissociated, and processed for flow cytometry analysis. Scatterplots on the right show typical distributions of cell suspensions from each treatment category. Wound samples show a characteristic influx of leukocytes (white open arrowhead) that is largely absent in uninjured tissue. While few B cells are typically present at either location, they can easily be detected in large numbers after experimental application (red arrow solid).

### Analysis of B cell-treated and control wound cell suspensions via flow cytometry

To assess changes in both B cells and cells of the wound environment between the various conditions and time points, samples were analyzed using flow cytometry. Our gating strategy and analysis of B cell-treated and control wound cell suspensions via flow cytometry is depicted in FIG. 4. Live cells were gated into 3 main categories: B cells (CD19+/B220+ lymphocytes), non-B cell leukocytes (CD140a-/B220- leukocytes) which included a mix of neutrophils, monocytes and macrophages, dendritic cells and T cells, and fibroblasts (CD140a+/B220-). These cell categories were evaluated for markers of activation and cytokine production.

### Dynamics of activation markers and key cytokines in B cells retrieved from the wound bed

The dynamics of activation markers and key cytokines in B cells retrieved from the wound bed after defined exposure intervals to the wound microenvironment are shown in FIG. 5. B cells were exposed in vivo to the wound niche or injected under uninjured skin (control equivalent location). Control B cells maintained on ice immediately after isolation for the same time duration are shown for comparison. After time intervals including 18 hours, 2 days, 4 days, and 10 days, the wounds were treated with Brefeldin A for 4 hours to induce retention of cytokines within cells. B cells were then retrieved by excising and dissociating the tissue, and further characterized by flow cytometry both for surface markers and intracellular cytokines. B cells exposed to the wound microenvironment transiently upregulate multiple immunomodulatory cytokines, peaking at 2 days post-application. Some immunomodulatory cytokines, including TGFβ and IL-6 remain elevated at 4 days, and IL-10 up to 10 days. N = 3-6 animals/group.

### Heatmap Analyses

A heatmap summary of the average values for each marker in B cells exposed to the wound microenvironment, subcutaneous control, or maintained on ice (no exposure) is found in FIG. 6. The dynamics of activation markers and key cytokines in the aggregate infiltrating non-B cell leukocytes in the wound are shown in FIG. 7. Overall, infiltrating leukocytes produced more anti-inflammatory cytokines IL-10, TGFβ, and IL-35, and less pro-inflammatory TNFα and IL-2 when B cells were present in the wound. This impact was most pronounced at 4 days post injury and B cell application and persisted up to 10 days. N = 3-6 animals/group.

A heatmap summary of the average values for each marker in infiltrating non-B cell leukocytes in the wound microenvironment, illustrating the pattern of increased anti-inflammatory cytokine (IL-10 and TGFb) production in the presence of B cells is shown in FIG. 8.

The dynamics of activation markers and key cytokines in the CD140a+ fibroblast population of the wound and subcutaneous tissue is shown in FIG. 9. Fibroblasts in the wound produced significantly more IL-10 and TGFβ at 10 days post-injury when wounds were exposed to B cells. Moreover, wound fibroblasts produce less of the pro-inflammatory cytokine TNFα when B cells were applied, both at 4 days and 10 days post-injury.

An additional heatmap summary of the average values for each marker in fibroblasts in wounds and subcutaneous tissue treated either with B cells or saline solution is shown in FIG. 10. Fibroblasts are among the most important sources of anti-inflammatory and pro-regenerative factors in wound healing and generate high levels of IL-10 and TGFβ regardless of treatment. Nevertheless, fibroblasts from wounds treated with B cells continued to produce higher levels of both IL-10 and TGFβ at 4 and 10 days post-injury, while in saline-treated wounds, the levels of these anti-inflammatory cytokines decreased. Interestingly, a significant effect of B cell application was observed in the reduction of pro-inflammatory cytokines in wound fibroblasts, including IL-6 and TNFα.

### TLR signaling as well as IL-10 production are necessary components of the regenerative function of exogenous B cells in wound healing

Functional TLR signaling as well as IL-10 production are necessary components of the regenerative function of exogenous B cells in wound healing as is shown in FIG. 11. Full-thickness excision wounds (illustrated here at day 6 of healing) were treated at day 0 with B cells lacking the common TLR-signaling adaptor myeloid differentiation factor 88 (MyD88), IL-10, or WT B cells as control. Saline was also included as internal control in each tested animal. While the WT B cells consistently accelerated the wound closure by 2-3 days in WT animals, MyD88-/- or IL-10-/- B cells showed no benefit for wound closure, similar to saline application.

### Example 2: B Cell Treatment Improves Outcome After TBI

This example illustrates that exogenously applied B cells significantly improved post-injury performance in a mouse TBI model.

Cerebral contusion causes neurological dysfunction mediated in part by inflammatory responses to injury. B lymphocytes are dynamic regulators of the immune system that have not been systematically studied in TBI. Using a mouse controlled cortical impact (CCI) model, we assessed a possible beneficial role of exogenously applied B cells on histopathological and functional outcome after TBI. Mice were injected intraparenchymally at the lesion site with 2×10⁶ mature naïve syngeneic splenic B cells, then subjected to CCI. Control CCI mice received equal numbers of T cells or saline, and sham-injured mice (craniotomy only) were given B cells or saline. Sham-injured groups performed similarly in motor and learning tests. Injured mice administered B cells showed significantly improved post-injury Rotarod, Y maze, and Morris water maze (MWM) performance compared to saline- or T cell-treated CCI groups. Moreover, lesion volume in mice treated with B cells was significantly reduced by 40% at 35 days post-TBI compared to saline and T cell controls, and astrogliosis and microglial activation were decreased. In vivo tracking of exogenous B cells showed that they have a limited lifespan of approximately 14 days in situ and do not appear to proliferate. The data suggest proof of principle that local administration of B lymphocytes represent a therapeutic option for treatment of cerebral contusion, especially when clinical management involves procedures that allow access to the injury site.

The study described below accordingly investigated the potential of mature naive B cells to protect against cognitive and histopathological deficits in the murine CCI TBI model. A single dose of B cells delivered by intraparenchymal injection at the time of injury was associated with significant improvements in hippocampus- and striatum-dependent behavioral tasks as compared to administration of splenic T cells or saline. The observed behavioral improvements were associated with significantly decreased lesion volume in animals treated with B cells, with sparing of hippocampal structures. In vivo tracking of the exogenously applied B cells after intraparenchymal injection showed that the cells have a limited survival of approximately 2 weeks in situ, indicating that they would represent a safe, feasible option for the treatment of acute and sub-acute contusion TBI.

### MATERIALS AND METHODS

The following materials and methods were utilized in this study.

**Animals:** All animal procedures were performed following the NIH Guide for Care and Use of Laboratory Animals and the Public Health Service Policy on Humane Care of Laboratory Animals. All protocols were approved by the Institutional Animal Care and Use Committee of Massachusetts General Hospital. Studies were performed in adult 12-14 week-old male C57BI6/J mice, weighing 25-32 g (Jackson Laboratories, Bar Harbor, ME). Male C57BI6/J and FVB-Tg(CAG-luc-GFP) L2G85Chco/J (all from Jackson Laboratories, Bar Harbor, ME) were used as isogeneic donors for B and T cell isolation. Animals were housed socially (4-5 individuals per cage) and maintained under standard laboratory care conditions, at temperatures ranging between 20-23°C, under a 12h light:dark cycle, with ad libitum access to food and acidified water. Animals were matched for age and randomly assigned to experimental conditions. To avoid bias, animals from different treatment arms were co-housed.

**Lymphocyte isolation:** Cell isolation was performed using negative immunomagnetic selection as described previously.¹⁶ Briefly, mouse spleens were collected in ice cold buffer containing 2% fetal bovine serum (FBS) and 1 mM ethylenediaminetetraacetic acid (EDTA) in phosphate-buffered saline (PBS). Spleens were dissociated mechanically through a 40 µm cell strainer and the splenocyte suspension was processed for negative B or T cell selection through immunomagnetic separation and retention of non-target cells, using commercially available cell isolation kits (STEMCELL Technologies, Inc., Vancouver, Canada), according to the manufacturer's instructions. The B cell isolation procedure was verified by flow cytometry analysis and typically resulted in an >98% pure population of mature naïve CD45R⁺/CD19⁺ B lymphocytes, with under 1% contamination with other leukocytes, although some residual erythrocytes may be present.¹⁶ Purified lymphocytes were re-suspended in sterile PBS at a concentration of 4 × 10⁵ cells/µl.

**Controlled cortical impact (CCI):** All surgical procedures, including the injury and the application of cells or saline, were performed by an experimenter blinded to the treatment conditions, who did not take part in the preparation of treatment doses for injection. Mice were anesthetized with 4.5% isoflurane (Baxter, Deerfield, IL) for 90 s in a mixture of 70% N₂O and 30% O₂ using a Fluotec3 vaporizer (Colonial Medical, Windham, NH) and placed in a stereotaxic frame. Anesthesia was maintained with 4.5% isoflurane. After a medial incision in the scalp, a craniotomy was made using a portable drill and 5-mm trephine over the left parieto-temporal cortex, and the bone flap was discarded. An ipsilateral intraparenchymal injection was delivered at approximately -1 mm from bregma on the anterior/posterior axis, +2 mm medial/lateral, at a depth of 3 mm through the left parietal cortex. A total of 5 µl of saline solution containing either 2 million B cells, 2 million T cells, or no cells, was injected using a 10-µl Hamilton syringe with a 26s gauge blunt tip needle (Hamilton Company, Franklin, MA). The selected cell dose has been optimized previously in a cutaneous injury model presenting similar lesion volume.¹⁶ Cell application was performed immediately before CCI to ensure correct and consistent injection of cells while the brain structure was intact. Mice were immediately thereafter subjected to CCI using a pneumatic cylinder with a 3-mm flat-tip impounder, at a velocity of 6 m/s, a depth of 0.6 mm and a 100-ms impact duration. Sham-injured mice underwent anesthesia, craniotomy, and intraparenchymal injection with equal numbers of B cells or saline, but no CCI injury. The craniotomy was left open and the skin was closed over the skull using 6-0 nylon sutures (Fisher Scientific, Waltham, MA).

**Behavioral testing schedule:** Behavioral testing was conducted during the light phase of the circadian cycle by experimenters blinded to the treatment conditions. Prior to each test, mice were acclimatized to the room for at least 30 min. Mice were tested in a battery of assays, according to the schedule described in FIG. 14. Vestibulo-motor ability was assessed by wire grip assay on days 1, 3, and 7 post injury. Rotarod testing was performed on days 7, 9, 10, 13, and 14 post injury. Animals were subjected to assessment of anxiety using an elevated plus maze assay on day 17 post injury. Morris water maze (MWM) testing was done on days 20, 21, 22, 23, and 24 after injury, with a probe test on day 27. On day 29 post injury, mice were subjected to the forced swim test to assay depression-like behavior, and on day 30 to the Y-maze, an assay for hippocampus-dependent working memory.

*Wire grip test:* Vestibulo-motor function was assessed using a wire grip test (Bermpohl et al. (2007) J Cereb Blood Flow Metab 27, 1806-1818). Mice were placed on a 45-cm-long metal wire suspended 45 cm above the ground and allowed to traverse the wire for 60 s. The latency to fall within the 60 s interval was measured, and a wire grip score was quantitated using a 5-point scale. Testing was performed in triplicate and an average value calculated for each mouse on each test day.

**Rotarod:** Mice were placed on an automated Rotarod apparatus (Harvard Apparatus, Holliston, MA) which accelerated from 4 to 40 r/min over 60 s. Maximum trial duration was 300 s, or until the mouse fell off the rotarod. Each mouse was assessed five times per day with 5 min rest intervals. The average latency to drop and the average r/min speed attained over the five trials was recorded for each day of testing.

**MWM:** The MWM was performed as previously described with minor modifications (Mannix et al. (2013) Ann Neurol 74, 65-75). Spatial learning was assessed at approximately the same time each day. Each mouse was subjected to seven hidden platform trials (one to two trials per day) using a random set of starting positions at any one of the four quadrants. One trial consisted of the average latency from each of the four starting positions. If a mouse failed to find the platform within 90 s, then it was placed on the platform for ~10 s. Probe trials were performed 24 h after the last hidden platform trial by allowing the mice to swim in the tank for 30 s with the platform absent, and recording the time spent in the target quadrant.

**Porsolt forced swim test:** Mice were placed in a cylindrical transparent glass tank of 30 cm (height) × 20 cm (diameter) filled with water (25°C) up to a height of 20 cm. A white Styrofoam box provided visual shielding on three sides. Mice were placed in the water for 6 min and swimming movements were recorded. Total active time (swimming, pawing/climbing the beaker wall) versus inactive time (passive flotation) was quantified for the last four minutes of the test.

**Y-maze spontaneous alternation test:** The Y maze test was conducted in an apparatus constructed of white opaque acrylic, consisting of three 40-cm long arms joined at 120° angles, with a wall height of 15 cm. Each arm was labeled with a different contrasting visual cue (black-on-white square, circle, star). Mice were placed in the center of the apparatus and allowed to explore the maze for 10 min. Their movements were recorded using a webcam positioned directly overhead and Photo Booth software (ANY-maze). Normal exploratory behavior in rodents involves a preference to enter a less recently visited arm of the maze (spontaneous alternation). An alternation score was calculated by dividing the number of three successive choices that included one instance of each arm by the total number of arm entries (i.e. opportunities for alternation). The apparatus was cleaned with 70% ethanol between trials.

**Elevated plus maze:** The apparatus consisted of two 130 × 8 cm platforms with a 8 × 8 cm square area at their intersection, elevated at 60 cm above ground. The closed arms of the platform had 10 cm walls, whereas the open arms had none. Each mouse was placed in the central area of the maze and video-recorded for 5 min. The apparatus was cleaned with 70% ethanol between trials. Video recordings were analyzed by ANY-Maze (Stoelting Co., Wood Dale, IL) software for mean speed and percent time in closed and open arms.

**IVIS imaging:** Splenic B cells were isolated from mice homozygous for the CAG-luc-eGFP L2G85 transgene, which show widespread expression of firefly luciferase and enhanced green fluorescence protein under the CAG promoter (Jackson Laboratories, Bar Harbor, ME). Approximately 5 million luciferase-expressing B cells in 5 µl PBS were injected into the left hemisphere of recipient WT C57B16/J mice, as described above. The mice were imaged using an IVIS Lumina II system (Caliper Life Sciences, Waltham, MA) on the day of the surgery and at regular intervals thereafter for a total of 4 weeks. For each imaging session, anesthesia was induced using 3% isoflurane in oxygen and maintained using 1 I/min of 2-3% isoflurane throughout the imaging session. To visualize luciferase activity, 100 µl of 30 mg/ml aqueous D-luciferin solution (Regis Technologies, Inc., Morton Grove, IL) was injected subcutaneously proximal to the injury site at least 6 minutes before imaging. Mice were imaged for 10 min, and identical parameters were maintained for every repeat imaging.

**Tissue sampling:** At 35 days after CCI and treatment, the mice were deeply anesthetized with ketamine (100 mg/kg) and xylazine (10 mg/kg), perfused transcardially with 10-15 ml of heparinized PBS to remove blood, and decapitated. The brains were rapidly extracted on ice, frozen in liquid nitrogen vapor, and stored at -80°C. For cryosectioning, the brains were embedded in M-1 embedding matrix (Thermo Fisher Scientific, Waltham, MA), and sectioned coronally at a thickness of 16 µm using a cryostat. Sections were collected at 500 µm intervals along the rostro-caudal axis, and thaw-mounted onto SuperFrost Plus Gold slides (Fisher Scientific, Waltham, MA).

**Immunohistochemistry:** Tissue processing for immunohistochemical analysis was performed as previously described (Sirbulescu et al. (2017) Wound Repair Regen 25, 774-791). Briefly, the sections were washed with PBS, then permeabilized and blocked by incubation for 1 hour at room temperature with PBS containing 5% bovine serum albumin, 5% fetal bovine serum, and 0.3% Triton X-100. Sections were then incubated overnight at 4°C with the following primary antibodies diluted in blocking solution: Alexa Fluor^{®} 594-conjugated rat anti-mouse CD45R/B220 (clone RA3-6B2; BioLegend, Inc., San Diego, CA), Alexa Fluor^{®} 488-conjugated mouse anti-mouse CD45.1 (clone A20; BioLegend, Inc., San Diego, CA), Alexa Fluor^{®} 488-conjugated mouse anti-glial fibrillary acidic protein (GFAP) (clone 2E1.E9; BioLegend, Inc., San Diego, CA), Alexa Fluor^{®} 647-conjugated rat anti-mouse CD68 (clone FA-11; BioLegend, Inc., San Diego, CA), and rabbit polyclonal anti-Ki67 (Abcam, Cambridge, MA). Unbound primary antibody was removed by 3 rinses in PBS. If unconjugated primary antibodies were used, antigenic sites were visualized by incubating the sections for 2 hours at room temperature with Alexa Fluor 488^{®}-conjugated F(ab')2- goat anti-rabbit IgG (Thermo Fisher Scientific, Waltham, MA), diluted 1:200 in blocking solution. Sections were counterstained by incubation with 2 µg/ml of 4', 6-diamidino-2-phenylindoledihydrochloride (DAPI; Sigma Aldrich). Antibody controls included incubation of the tissue sections with isotype antibodies and omission of the primary antibody when a secondary antibody was used for visualization. No unspecific signal was detected in the control samples. Stained tissue sections were imaged using a Zeiss LSM 710 laser scanning microscope (Carl Zeiss), and confocal images were collected using Zen software (Carl Zeiss).

**Lesion volume measurement:** Sections were stained with hematoxylin and high-resolution overview photographs of the slides were collected. Morphometric image analysis in ImageJ (NIH, Bethesda, MD) was used to determine the area of each hemisphere. For each section, the area of the injured hemisphere (left) was subtracted from the area of the uninjured hemisphere and the difference was multiplied by 0.5 to obtain the volume of brain tissue loss, expressed in mm³.

**Image analysis:** For unbiased quantification of GFAP and CD68 immunolabeling, confocal images collected around the lesion site, including both the medial and lateral aspects of the lesion area (n = 4 fields of 1400 × 1400 µm per animal) were analyzed using standard functions in MATLAB (The MathWorks, Inc., Natick, MA). For each image, regions of interest for analysis were defined semiautomatically through morphological closing, morphological opening, and interactive filling of a binary mask generated through the application of a minimum intensity threshold of 10 to the maximum intensity projection across all imaged channels. Background levels were determined through image smoothing using a 3x3 pixels moving average, followed by morphological opening using a 10-pixel radius structuring element, separately for each channel. Foreground objects were identified as sets of pixels with intensities exceeding the corresponding background levels by more than 25 for GFAP, respectively 50 for CD68 immunolabeling. For each analyzed marker, the relative area labeled was calculated as the fraction of pixels within the region of interest marked as foreground, while the mean labeling intensity was calculated as the average intensity of all foreground pixels found within the region of interest. All image analyses were performed by an experimenter blinded to the treatment conditions.

**Statistical analysis:** Prior to statistical testing, all datasets were assessed for normal distribution using the D'Agostino & Pearson normality test and were found to pass (p > 0.2). The statistical significance of differences between experimental groups for repeated behavioral tests, including wire grip assay, Rotarod, MWM hidden and visible platform, and elevated plus maze, was assessed using two-way (treatment × time) repeated-measures ANOVA (with trial/timepoint as a repeated-measures factor) with matched subjects, followed by post-hoc multiple comparisons using the Tukey or Sidak method. Single-timepoint measurements, including MWM probe, Y maze, forced swim, as well as histology comparisons, were assessed using one-way ANOVA followed by Tukey's multiple comparisons test. All reported descriptive statistics are estimated marginal means ± standard error of the mean (SEM). All statistical analyses were conducted using GraphPad Prism 7 (GraphPad Software, Inc., La Jolla, CA). P < 0.05 was considered statistically significant.

### RESULTS

### B lymphocyte application at the time of injury improves cognitive functional recovery after CCI

A total of 63 mice finished the study, with 1 death. In the rotarod assay, intraparenchymal treatment with B lymphocytes had a significant protective effect (FIG. 15A). While all injured groups performed worse than sham-lesioned animals, injured mice treated with B cells showed better performance than injured T cell- or saline-treated groups (p < 0.01 for group), and their performance did not differ from that of B-cell treated shams. Interestingly, consecutive trials showed a continued improvement in performance in the CCI + B cells group, as well as in both sham-lesioned control groups, suggesting that procedural learning occurred in these groups. There was no significant difference between the B cell-treated CCI group and sham-injured mice, except for trial 3, in which B cell-treated sham-lesioned mice performed better (p < 0.01). By contrast, CCI mice treated intraparenchymally at the time of injury with either saline or 2 million T cells showed minimal day-to-day improvement in performance and had significantly lower latencies to fall as compared to either B cell-treated CCI injured or sham-injured animals (p < 0.0001). There was no statistically significant difference between CCI injured groups that received T cells or saline.

In the wire grip test, all injured groups performed worse than shams, however no group differences were observed among B cell-, saline-, or T cell-treated CCI groups (FIG. 15B).

In the MWM (FIGs. 16A-D), sham-injured mice performed significantly better in the hidden platform trials than all CCI groups, indicating a robust effect of CCI (p < 0.001 for group), with no differences noted among the sham-injured groups. Among injured groups there was a highly significant effect of treatment (p < 0.0001) with a significant treatment × trial interaction effect (p = 0.02) in B cell-treated vs. saline-treated mice on trial 7 (p = 0.016, FIG. 16A). The rate of performance improvement over the course of the 7 hidden-platform trials, as estimated by the difference in average time-to-platform between trial 1 and trial 7, was significantly higher in injured animals that received B cells as compared to saline controls (p < 0.01). No significant differences among any of the groups were observed in visible platform trials (FIG. 16B). In probe trials, injured B cell-treated mice performed similar to sham groups (FIG. 16C). By contrast, CCI injured mice that received either T cells or saline performed worse than shams (p < 0.05). Swim patterns of injured B cell treated mice demonstrated evidence for spatial search strategies similar to those of sham injured groups whereas search strategies were non-spatial in injured T cell and saline-treated groups (FIG. 16D).

In Y-maze testing (FIG. 16E), CCI injured mice that received B cells intraparenchymally had alternation scores of 76.65 ± 2.46, significantly higher than injured animals treated with either T cells (46.86 ± 2.41) or saline (38.92 ± 2.56; p < 0.0001) and similar to sham-injured B cell-treated mice. There was no significant difference between any of the injury or treatment groups with respect to the total number of maze arm entries (p > 0.13) or the total distance covered (p > 0.07).

In the elevated plus maze (FIG. 17A), there was no significant difference between any of the treatment groups in the time spent in the open arms (p > 0.84) or the center starting area (p > 0.94). There was a significant treatment × location interaction (p < 0.05). Among the injured groups, mice that received B cells spent significantly more time in the closed arms of the maze as compared to animals that received an equal number of T cells (p < 0.05).

Sham and injured groups did not differ amongst each other in the forced swim test suggesting that the lesion paradigm employed here does not affect depression-like behavior as measured by this assay (FIG. 17B).

### B lymphocyte application at the time of injury is associated with reduced lesion volume and glial scarring at 35 days post CCI

To assess whether the behavioral improvements observed in association with B cell treatment have a neuropathological correlate, brains from all animals subjected to behavioral assessments were collected on day 35 after injury for histological examination (FIG. 18). Analyses of brain tissue damage in the CCI groups showed cavitation of the lesioned area in all mice subjected to CCI, whereas sham-lesioned groups showed no loss of brain tissue (FIG. 18A). Quantitative analysis of the lesion volume across all groups (FIG. 18B) showed a significant effect of CCI as expected (p < 0.0001). B cell-treated CCI mice had significantly less brain tissue loss as compared to injured groups treated with saline (p < 0.001) or T cells (p < 0.0001). There was no significant difference in lesion volume between the CCI injured groups that received T cells or saline. Analysis of the lesion area distribution in consecutive sections throughout the brain (FIG. 18C), showed that the most pronounced difference between the treatment conditions was observed in the posterior 2/3 of the lesion, at levels where it involved mostly the hippocampus. Indeed, targeted volumetric measurements showed that B cell-treated CCI mice had a significantly larger proportion of spared hippocampal tissue in the injured hemisphere as compared to either saline- or T cell-treated CCI controls (p < 0.0001; FIG. 18D).

To assess the effect of B cell treatment on long-term reactive responses in the injured brain, including astrogliosis and microglial activation, tissue sections collected at 35 days after injury were immunolabeled for GFAP and the activation-associated marker CD68, respectively. Unbiased analysis of confocal images collected from locations adjacent to the cavitation lesion formed at the site of the initial CCI injury showed a significant effect of B cell treatment delivered at the time of injury, as compared to saline- and T cell-treated controls (FIG. 19). In B cell-treated animals, glial scarring, as indicated by intense GFAP immunolabeling and extensive astrocyte hypertrophy was significantly reduced, as was the immunolabeling against the microglial activation marker CD68 (FIGs. 19E, F).

### B lymphocytes do not proliferate in situ and have a limited lifespan of approximately 2 weeks after application

To determine whether exogenously delivered B cells persist in vivo after intraparenchymal injection we used purified B cells that constitutively expressed firefly luciferase under the CAG promoter. Since luciferase has a short biological half-life, it must be continuously produced for detection. When luciferase-expressing cells die, the signal is quickly lost, thus this assay can be used to determine the long-term survival of experimentally-introduced B cells (Zinn et al. (2008) ILAR J 49, 103-115). As only exogenous, live B cells will generate light signal, this method allows the non-invasive tracking of cell viability and persistence over time, down to a few hundred cells. Results showed that the injected B cells were clearly detectable immediately after application in all animals examined (FIG. 20A). Quantitative analysis of the total photon flux in the head region indicated that the active enzymatic activity within the applied cells initially showed a moderate increase, peaking at days 3-7, and then rapidly declined after day 7, reaching undetectable levels by day 17 post injection (FIG. 20B). These results support the view that exogenous B cells have a limited life span of approximately 2 weeks in situ.

To investigate whether the introduced B cells may proliferate in situ, thereby potentially establishing a more long-lived population within the brain parenchyma, coronal brain sections through the injection site were collected and immunolabeled for B cell and proliferation markers. Labeling B cells ex vivo using toluidine blue prior to injection showed that the cells only distributed within a radius of approximately 1 mm around the injection site after delivery (FIG. 21A). Confocal imaging confirmed that the injected B cells remain well localized at the application site up to 4 days after delivery, with some dispersal throughout the injury (FIG. 21D). Immunolabeling for the proliferation marker Ki67 showed that B cells did not express this marker at the 0-day or 4-day timepoints, while neighboring non-B cells showed high levels of Ki67 immunopositivity 4 days after injury in all of the animals examined (n = 4 animals per time point; FIG. 21D, E). This finding was consistent with the fact that no B cells could be observed at 35 days post application in tissue sections immunolabeled for CD45R (B220) and CD45.1 in either injured or sham-lesioned animals (data not shown). While in CCI injured animals at day 35 the original injection site is disrupted by cavitation, in sham-lesioned controls it can be easily detected due to gliosis around the injury caused by the needle (FIG. 21F). By 35 days, no B220-positive B cells were found at the injection site in B cell-treated shams (FIG. 21F, G).

### Conclusions

This study describes, for the first time to our knowledge, the use of a direct application of B lymphocytes in a preclinical TBI model to modulate structural and functional outcome after injury. We found that a single intraparenchymal delivery of purified (>95%) mature, naïve B cells at the time of CCI can significantly reduce post-injury learning and memory deficits and reduce brain tissue loss. The results suggest heretofore unknown protective effects of endogenous B cells in a cerebral contusion model.

The CCI injury model produces highly reproducible lesions as well as very low mortality rates (Xiong et al., (2013) Nat Rev Neurosci 14, 128-142). While the impact is delivered to the cortical surface, with the dura intact, the neuropathological consequences of the injury are typically extensive, and include cortical, hippocampal, and thalamic degeneration (Id.) These pathologies are associated with long-term cognitive deficits and alterations in emotional behavior (Id.). In the present CCI injury paradigm, as the impact force was applied directly to the cortex, this region reliably and invariably degenerated in all animals, regardless of treatment condition, while differences between therapeutic conditions were observed in the sparing of subcortical structures, particularly the hippocampus. The observed behavioral benefits of B cell treatment were also well correlated with functions supported by these structures, suggesting a connection between the localization of the introduced lymphocytes, the sparing of tissues around the injection site, and functional neuroprotection. Supporting this hypothesis, in the present model, B lymphocytes were injected approximately 1 mm posterior from bregma and remained mostly localized between the caudoputamen and the hippocampus (Lein et al. (2007) Nature 445, 168-17).

In the present study, confocal imaging of tissue sections collected from the site of CCI showed that B cells were clustered around the initial injection site, with moderate distribution throughout the injured tissue by day 4 after injury and application. These results illustrate that intraparenchymally injected B cells likely remain at the lesion site for their lifespan in situ. While the molecular mechanisms underlying the observed neuroprotective effects of B cells are not fully understood, it is likely that diffusible factors originating from the cells are involved, reaching distal areas beyond the strict localization of the applied cells. This hypothesis is supported by the observations that in animals treated with B cells at the time of CCI, the lesion volume was reduced significantly, and potentially detrimental reactive phenomena, including astrogliosis and microglial activation were significantly decreased at the injury site, as compared to controls.

B cells accordingly may be used as a therapeutic strategy for patients with cerebral contusion. Unlike any other existing cell-based therapies, B cells can be readily obtained from peripheral blood or other blood bank products, an important advantage for the development of a rapid off-the-shelf therapeutic agent. Indeed, a rapid, minimally-manipulated, autologous B cell therapy would be highly translatable into a clinical setting. This is especially true in the case of severe brain lesions, where surgery is often performed to remove hematomas or penetrating bone fragments, and either intraparenchymal or intraventricular catheters are placed to monitor intracranial pressure, (Stocchetti et al. (2017) Lancet Neurol 16, 452-464; Galgano et al. (2017) Cell Transplant 26, 1118-1130) thus providing a convenient route of administration for B cells into injured brain.

Unlike other cell types used therapeutically, such as stem cells, B lymphocytes are mature, terminally-differentiated cells, with a naturally limited lifespan of 5-6 weeks in vivo. Their application in the disrupted microenvironment of a brain contusion is expected to lead to elimination of the transplanted cells after even less time. This is advantageous because longer survival of transplanted cells could represent a significant safety concern, particularly considering that the microenvironment of the central nervous system contains a number of B cell-trophic factors. We used in vivo visualization of luciferase-expressing B cells to monitor the presence and persistence of metabolically-active transplanted cells over time. We observed that the cell signal rapidly declined after day 7, reaching undetectable levels by day 17. In the context of CCI, there was an initial moderate increase in luciferase signal intensity between 3 and 7 days after intraparenchymal injection, potentially indicating that the transplanted cells underwent a period of acclimation and/or stimulation in the local microenvironment of the CCI injury. It is unlikely that the signal intensity increase was due to cell proliferation in situ, since immunohistochemical examination of the injection site either immediately or up to 4 days after B cell administration and CCI showed that the introduced B cells do not express markers of cell proliferation. Indeed, B cells were not observed to proliferate after application to a wound, although they did enhance the proliferation of adjacent cells.

This study describes a first proof-of-principle observation that mature peripherally isolated B cells could represent a safe, rapid, and effective cell-based therapeutic strategy for the acute and sub-acute treatment of contusion TBI, for which no therapeutic options to improve neurological outcome exist currently.

### Example 3: Evaluation of B Cell Immunotherapy in the SOD1-G93A Mouse Model of ALS

This example illustrates the safety and efficacy of intravenous (i.v.) B cell administration in a standard murine model of ALS, the SOD1^{G93A} mouse.

Male and female transgenic SOD1^{G93A} mice as well as equal numbers of sex-matched non-carrier controls (n = 32/condition) were treated starting at week 10 of life (day 72) with 5×10⁶ B cells in saline (or saline control) on a weekly schedule for a total of 10 weeks. We found that B cell treatment delayed symptom onset (p<0.0001), as indicated by reaching a peak weight, and significantly extended survival (p<0.05) in SOD1^{G93A} mice. Treatment with B cells was associated with a significant reduction in the relative numbers of injured/degenerating motor neurons in the lumbar spinal cord (p<0.05) at endpoint, even as total numbers of motor neurons were not changed with treatment. B cell treatment was not associated with any observable (behavioral and phenotypic) detrimental effects in identically-treated non-transgenic control wild-type littermates, which continued to gain weight over the duration of the repeated treatment. Taken together, these findings demonstrate that B cell therapy may provide a viable method for mitigating neuroinflammation in ALS.

ALS is a fatal disease characterized by the progressive degeneration of both upper motor neurons in the cerebral motor cortex, and lower motor neurons in the brainstem and ventral horn of the spinal cord. To date, there are no cures for ALS, highlighting an urgent and unmet need in the field.

### STUDY DESIGN

To assess the efficacy of B cell immunotherapy in ALS, we used the well-established B6.SOD1^{G93A} transgenic mouse model of ALS according to the published guidelines on the design and interpretation of results (Galgano (2017) Cell Transplant 26, 1118-1130; Nordstrom et al. (2014) Ann Neurol 75, 374-381). Mice with transgenic expression of a G93A mutant form of human SOD1 exhibit a phenotype similar to ALS in humans. They become progressively paralyzed in one or more limbs, with paralysis due to loss of motor neurons from the spinal cord. Transgenic mice also have a shorter life span. Neurodegenerative symptoms typically start appearing around 12-14 weeks of age and the mice die at approximately 20-24 weeks of life. This model thus shows rapid, aggressive progression of the disease. The model is very stable and reproducible, allowing the assessment of therapeutic options for this neurodegenerative disorder.

A total of 15-17 females and males (per recommended guidelines in the field (Id.)), as well as equal numbers of sex-matched non-littermate non-carrier controls were used for each experimental condition (treatment) throughout the study (Table 1). Starting at week 10 of life (day 72), all animals received a total of 10 weekly intravenous infusions of B cells (or saline control), delivered via retro-orbital injection (FIG. 22). For feasibility, the study was performed in 2 overlapping cohorts as described in Table 1 (Overview of animals used by genotype, sex, and treatment for each cohort).

**Table 1**

| | **Transgenic B6.SOD1^{G93A}** | | | | **Noncarrier control (Wt)** | | | |
|---|---|---|---|---|---|---|---|---|
| | **Males** | | **Females** | | **Males** | | **Females** | |
| | **Saline** | **B cells** | **Saline** | **B cells** | **Saline** | **B cells** | **Saline** | **B cells** |
| **Cohort 1** | 11 | 12 | 11 | 12 | 9 | 10 | 10 | 11 |
| **Cohort 2** | 5 | 5 | 6 | 4 | 7 | 6 | 5 | 5 |
| **Total** | **16** | **17** | **17** | **16** | **16** | **16** | **15** | **16** |

### MATERIALS AND METHODS

The following materials and methods were used in this study.

**Animals:** B6SJL-Tg(SOD1*G93A)1Gur/J (SOD1-G93A) transgenic mice were purchased from The Jackson Laboratory (Bar Harbor, ME; Stock No: 002726). This founder line (often referred to as G1H) is reported by Jackson Laboratory to have high SOD1 transgene copy number. All animals are individually genotyped by Jackson Laboratory prior to shipping and only heterozygous animals with high SOD1 transgene copy numbers (upper third of the distribution) are commercialized. Control animals are littermates without the SOD1 transgene (Noncarrier).

Donor animals for B cell isolation were C57BL/6J mice also purchased from the Jackson Laboratory (Stock No: 000664).

All animal procedures were performed following the NIH Guide for Care and Use of Laboratory Animals and the Public Health Service Policy on Humane Care of Laboratory Animals. All protocols were approved by the Institutional Animal Care and Use Committee of Massachusetts General Hospital, protocol #: 2019N000004.

**B cell isolation:** All cell isolation procedures were performed under sterile conditions, in a clean biosafety cabinet, and the resulting cell suspension was delivered in sterile phosphate-buffered saline (PBS). Cells were isolated and purified from the spleens of C57BL6 wild-type donor animals, sharing half of the genetic background of the recipient (similar to a sibling). Spleens were dissociated into a splenocyte suspension, and commercially available kits (Miltenyi B Cell Isolation Kit, mouse; 130-095-873, Miltenyi Biotec) were used to isolate all B cells by negative immunomagnetic selection as in our previously published protocols (DeKosky et al. (2013) Nat Rev Neurol 9, 192-200; Stocchetti et al. (2017) Lancet Neurol 16, 452-464). The resulting cells are >98% CD19+ B cells, and typically over 85-90% CD19+/B220+/lgM+/lgD+, including approximately 5% CD138+ plasma cells, and <1% other cell populations, as confirmed after isolation through flow cytometric analysis (DeKosky et al. (2013) Nat Rev Neurol 9, 192-200). This represented the naïve B cell fraction (Treatment), and it was infused into animals the same day, after isolation.

**Treatment:** Starting at week 10 of life (day 72), all animals received a total of 10 weekly intravenous infusions of B cells (or saline control), delivered via retro-orbital injection. The animals were anesthetized with 3% isoflurane in oxygen and a 100 µl bolus of saline containing 5 million naïve B cells (Treatment) or no cells (saline control) was injected into the retro-orbital venous sinus. Eye ointment was then applied to the treated eye. This method of administration was selected because it has a considerably lower risk of failure as compared to tail vein injection for cell transplantation, particularly with repeated administration.

**Safety Assessment:** Three times per week the health status of all the animals was assessed for potential detrimental effects of the treatment as per IACUC guidelines (weight loss, apathy, poor body condition, ruffled fur, hunched posture).

**Efficacy Assessment:** Weight and neurological scores (NeuroScore) were assessed twice weekly by an experimenter blinded to the treatment conditions, and were used to assess disease progression, as described e.g. in Hatzipetros, T. et al. (2015) J. Vis. Exp. (104), e53257, doi:10.3791/53257; Mashkouri et al. (2016) Neural Regen Res 11, 1379-1384 :
NeuroScore 0 (Pre-symptomatic): When the mouse is suspended by the tail, the hindlimb presents a normal splay i.e., it is fully extended away from the lateral midline and it stays in this position for 2 sec or longer. When the mouse is allowed to walk, normal gait is observed.
NeuroScore 1 (First symptoms): When the mouse is suspended by the tail, the hindlimb presents an abnormal splay, i.e., it is collapsed or partially collapsed towards lateral midline OR it trembles during tail suspension OR it is retracted/ clasped. When the mouse is allowed to walk, normal OR slightly slow gait is observed.
NeuroScore 2 (Onset of paresis): When the mouse is suspended by the tail, the hindlimb is partially OR completely collapsed, not extending much. (There might still be joint movement). When the mouse is allowed to walk, the hindlimb is used for forward motion however the toes curl downwards at least twice during a 90 cm walk OR any part of the foot is dragging along. When the mouse is placed on its left AND right side, it is able to right itself within 10 sec from BOTH sides.
NeuroScore 3 (Paralysis): When the mouse is suspended by the tail, there is rigid paralysis in the hindlimb OR minimal joint movement. When the mouse is allowed to walk there is forward motion however the hindlimb is NOT being used for forward motion. When the mouse is placed on its left AND right side, it is able to right itself within 10 sec from BOTH sides.
NeuroScore 4 (Humane end-point): When the mouse is suspended by the tail, there is rigid paralysis in the hindlimbs. When the mouse is allowed to walk, there is no forward motion. When the mouse is placed on its left AND right side it is NOT able to right itself within 10 sec from EITHER side. i.e., absence of righting reflex.

Weight was used as a reliable and unbiased assessment of disease progression. The appearance of disease onset was retrospectively determined using the age of maximal body weight which is a reliable and objective measure of muscle denervation onset, as previously described (Turner et al. (2014) Neurobiol Aging 35, 906-915.). Histology: At euthanasia, the lumbar spinal cord was collected from all animals, preserved in fixative solution, and processed for histology. Spinal cords were sectioned longitudinally in the horizontal plane, at a thickness of 10 µm and stained with hematoxylin and eosin (H&E) to visualize motor neurons in the ventral horns. Motor neurons, easily identifiable based on large size and distinctive morphology, were counted in 3-6 regions of interest of at least 500 x 500 µm, randomly collected from 2-3 longitudinal sections per animal. We also quantified separately healthy motor neurons (large, rounded cell body; nuclei with single prominent nucleoli; Nissl substance present; see FIG. 27A) and injured/degenerating motor neurons (shrunken cell body, hyperbasophilia, strong aggregation of nuclear chromatin, pyknotic nucleus; see FIG. 27B). All counts were performed by an experimenter blinded to the treatment conditions.

### RESULTS

The following results summarize data collected from the full animal study. The study ended at post-natal day 150, when the last of the transgenic SOD1 animals was euthanized.

### Safety

No observable (behavioral and phenotypic) detrimental effects (weight loss, apathy, poor body condition, ruffled fur, hunched posture) were found in control noncarrier animals that received naïve B cells (at a dose of approximately 200 million cells/kg). Control animals continued to gain weight throughout the study, independent of B cell treatment (FIG. 23). Moreover, in transgenic SOD1-G93A animals, no such detrimental effects that could be attributed to the cell infusion treatments were observed in the period before symptom onset (days 72-90 of treatment).

### Efficacy of treatment on symptoms of ALS progression

**a. Peak weight** (shown in FIG. 24). Peak weight was defined as the time point after which the measured weight of an individual animal showed a continual decrease. This information was used to perform survival analyses, where time of peak weight represents "survival". Only transgenic SOD1-G93A animals were used for this analysis, since control noncarrier mice do not show weight loss.
   Peak weight (i.e. onset of symptoms, associated with weight decline) was delayed on average by approximately 28 days in the naïve B cell treatment condition as compared to controls treated with saline (p < 0.0001, one-way ANOVA with Sidak post-hoc correction for multiple comparisons)(see Turner et al., Neurobiol. Aging 35, 906-915 (2014)).
**b. Neurological score assessment** (shown in FIG. 25). For the present analysis, disease onset was defined as the time point at which animals had received a neuroscore of 1 for 3 consecutive assessments, and no decrease in neuroscore followed. A progressive increase in neurological score over time was observed in all animals, however B cell treatment was associated with a slower rate of progression (FIG. 25).
**c. Survival analysis** (shown in FIG. 26). As described above, animals undergoing full paralysis, which were unable to right themselves within 10 seconds of being placed on either side, were categorized as having a Neuroscore of 4, and humanely euthanized. These animals are counted as having died due to ALS. Analysis of death as a consequence of disease progression demonstrated a significant overall survival benefit associated with intravenous administration of naïve B cells (p = 0.0286, one-way ANOVA with Sidak post-hoc correction for multiple comparisons; FIG. 26).
**d. Histological analysis of spinal cord motor neurons** (shown in FIG. 27). Processing and examination of all histological samples was performed by an experimenter blinded to the treatment conditions. As expected, a very significant decrease in the overall numbers of motor neurons in the ventral horns of the lumbar spinal cord was observed in transgenic SOD1 animals as compared to WT controls. While no significant difference was observed between the B cell-treated and control transgenic SOD1 animals in the total number of neurons found in the spinal cord, a significantly lower percentage of these motor neurons were found to be dead or dying in B cell treated animals as compared to saline control.

### Conclusions

In this study in the transgenic SOD1-G93A mouse model of ALS, intravenous injections of purified allogeneic mature naïve B cells administered weekly for 10 consecutive weeks to mice were associated with (i) a statistically significant 28-day delay in symptom onset, (ii) a significant extension of survival, and (iii) a significant reduction in impaired, dead or dying neurons present in the lumbar spinal cord of treated animals as compared to saline-treated controls. No observable detrimental side effects associated with the treatment were noted in wild-type or transgenic animals that received B cell injections at a dose of 200,000 cells/gram (or 200 million B cells/Kg).

### Example 4. Administration of B cells to treat Parkinson's' disease

A composition including a therapeutically effective amount of B cells, such as any of the compositions described herein, may be administered to a subject having Parkinson's' disease. Treatment of Parkinson's' disease may be evaluated using the methods described herein by administering therapeutic B cells (e.g., B_{reg} cells) to an appropriate animal model for Parkinson's' disease (see, e.g., Bobela W. et al. Overview of mouse models of Parkinson's disease. Curr Protoc Mouse Biol. (2014)) and monitoring the therapeutic efficacy according to methods known to those of skill in the art. Methods for monitoring the response include assessment of motor function, pain, neuroinflammation, and death of nigral neurons (see, e.g., Peng Q. et al. The Rodent Models of Dyskinesia and Their Behavioral Assessment. Front Neurol. (2019)).

Responsiveness to treatment may be monitored by a decrease in the rate of progression of the disease (e.g., a decrease in the rate of progression as measured by the severity of symptoms associated with Parkinson's' disease). Alternately, responsiveness to treatment may be monitored by determining the level of a molecular marker of disease progression associated with neurodegenerative disease, such as, T-tau (total tau), P-tau (hyperphosphorylated tau), Aβ42 (amyloid beta 42), the ratio of Aβ42/Aβ40, YKL-40 (Chitinase-3-like protein 1), VLP-1 (visinin-like protein 1), NFL (neurofilament light), pNFH (phosphorylated neurofilament heavy subunit), Ng (neurogranin) and UCH-L1 (ubiquitin C-terminal hydrolase), TDP-43 (TAR DNA-binding protein 43), decreased α-synuclein and/or decreased levels of 3,4-dihydroxyphenylacetate (see, e.g., Robey and Panegyres. Cerebrospinal fluid biomarkers in neurodegenerative disorders. Future Neurol. 14(1). (2019)).

### Example 5. Administration of B cells to treat additional neurodegenerative diseases

Other neurodegenerative diseases may be evaluated using the methods described herein by administering B cells (e.g., Breg cells) to an appropriate animal model. Additional neurodegenerative diseases include Alzheimer's disease, chronic traumatic encephalopathy (CTE), frontotemporal dementia, Huntington's disease, infantile neuroaxonal dystrophy, progressive supranuclear palsy, Lewy body dementia, spinocerebellar ataxia, spinal muscular atrophy, and motor neuron disease

Exemplary animal models for the study of such neurodegenerative disease have been described in the art, for example in:
Alzheimer's disease (see, e.g., Esquerda-Canals G. et al. Mouse Models of Alzheimer's Disease. J Alzheimers Dis. (2017));
Chronic traumatic encephalopathy (CTE) (see, e.g., Dapul HR, et al. Concussive injury before or after controlled cortical impact exacerbates histopathology and functional outcome in a mixed traumatic brain injury model in mice. J Neurotrauma. 30(5):382-91 (2013)); and
Huntington's disease (see, e.g., Farshim PP, et al. Mouse Models of Huntington's Disease. Methods Mol Biol. (2018)).

The responsiveness to treatment may be monitored by a decrease in the rate of progression of the disease (e.g., a decrease in the rate of progression as measured by the severity of symptoms associated with the neurodegenerative disease). Alternately, responsiveness to treatment may be monitored by determining the level of a molecular marker of disease progression associated with neurodegenerative disease, such as, a molecular marker of disease progression provided in Example 4.

### Example 6. Administration of B cells to treat inflammatory or immune diseases

B cells described herein may be administered to treat inflammatory or immune disorders, such as, cystic fibrosis, cardiovascular disease (e.g., coronary artery disease or aortic stenosis), keratoconus, keratoglobus, osteoarthritis, osteoporosis, pulmonary arterial hypertension, retinitis pigmentosa, or rheumatoid arthritis. Treatment of these inflammatory or immune disorders may be evaluated using the methods described herein by administering therapeutic B cells (e.g., Breg cells) to an appropriate animal model and monitoring the therapeutic efficacy according to methods known to those of skill in the art.

Exemplary animal models for the study of such inflammatory or immune disease have been described in the art, for example in:
Cystic fibrosis (see, e.g., Dreano, E. et al. Characterization of two rat models of cystic fibrosis-KO and F508del CFTR-Generated by Crispr-Cas9. Animal Model Exp Med. 2(4):297-311 (2019));
Cardiovascular disease (Goodchild, T.T. et al. Bone marrow-derived B cells preserve ventricular function after acute myocardial infarction. JACC Cardiovasc Interv. 2(10):1005-16 (2009));
Keratoconus (see, e.g., Tachibana M. et al. Androgen-dependent hereditary mouse keratoconus: linkage to an MHC region. Invest Ophthalmol Vis Sci. 43(1):51-7 (2002));
Osteoarthritis (see, e.g., Kuyinu. E.L. et al. Animal models of osteoarthritis: classification, update, and measurement of outcomes. J Orthop Surg Res. 11:19 (2016));
Osteoporosis (see, e.g., Komori T. Animal models for osteoporosis. Eur J Pharmacol. 759:287-94 (2015));
Pulmonary arterial hypertension (see, e.g., Sztuka K. and Jasińska-Stroschein M. Animal models of pulmonary arterial hypertension: A systematic review and meta-analysis of data from 6126 animals. Pharmacol Res. 125(Pt B):201-214 (2017));
Retinitis pigmentosa (see, e.g., Tsubura A. et al. Animal models for retinitis pigmentosa induced by MNU: disease progression, mechanisms and therapeutic trials. Histol Histopathol. 25(7):933-44 (2010)); and
Rheumatoid arthritis (see, e.g., Asquith D.L. et al. Animal models of rheumatoid arthritis. Eur J Immunol. 39(8):2040-4 (2009)).

### Other Embodiments

From the foregoing description, it will be apparent that variations and modifications may be made to the invention described herein to adopt it to various usages and conditions. Such embodiments are also within the scope of the following claims.

Some aspects of the present disclosure are summarized in the following items:
1. A method of treating a neurodegenerative disease in a subject in need thereof, the method comprising administering to the subject a therapeutically effective amount of isolated B cells.
2. The method of item 1, wherein the neurodegenerative disease is selected from amyotrophic lateral sclerosis (ALS).
3. The method of item 1, wherein allogeneic B cells are administered.
4. The method of item 1, wherein autologous B cells are administered.
5. The method of item 1, wherein xenogeneic B cells are administered.
6. The method of item 1, further comprising administering a second therapeutic composition.
7. The method of item 6, wherein the second therapeutic composition is Edaravone or Riluzole.
8. The method of item 6, wherein the second therapeutic composition is an immunomodulatory composition.
9. The method of item 1, wherein the B cells are mature naïve B cells.
10. The method of item 1, wherein B cells are stimulated ex vivo.
11. The method of item 1, wherein the B cells are stimulated with a Toll-like receptor (TLR) agonist.
12. The method of item 1, wherein the B cells are Breg cells.
13. The method of item 12, wherein the Breg cells express immunomodulatory cytokine IL-10.
14. The method of item 12, wherein the Breg cells comprise at least 80% CD19+ B cells.
15. The method of item 12, wherein the Breg cells comprise less than 10% CD138+ plasma B cells.
16. The method of item 1, wherein the B cells are formulated to be administered locally.
17. The method of item 1, wherein the B cells are formulated to be administered systemically.
18. The method of item 1, wherein the B cells are formulated to be administered intravenously, intraarterially, subcutaneously, intrathecally, or intraparenchymally .
19. The method of item 1, wherein the B cells are administered once daily, once weekly, twice weekly, once every 14 days, once monthly, once every two months, once every three months, once every four months, once every five months, once every six months, or once yearly.
20. The method of item 1, wherein the therapeutically effective amount comprises at least 0.5 x 107 B cells per administration.
21. The method of item 1, wherein the therapeutically effective amount comprises at least 1 x 108 B cells per administration.
22. The method of item 1, wherein the therapeutically effective amount comprises at least 2 x 108 B cells per administration.
23. The method of item 1, wherein the therapeutically effective amount comprises at least 1 x 109 B cells per administration.
24. A method of treating a subject having a traumatic brain injury (TBI), comprising administering to the subject a therapeutically effective amount of isolated B cells.
25. The method of item 24, wherein TBI results from a head injury, or a cerebral contusion.
26. The method of item 24, wherein the subject suffers from one or more of a number of physical, cognitive, social, emotional and/or behavioral disorders.
27. The method of item 24, wherein allogeneic B cells are administered.
28. The method of item 24, wherein autologous B cells are administered.
29. The method of item 24, wherein xenogeneic B cells are administered.
30. The method of item 24, additionally comprising administering a second therapeutic composition.
31. The method of item 24, wherein the second therapeutic composition is an antibiotic or a corticosteroid.
32. The method of item 24, wherein the B cells are mature naïve B cells.
33. The method of item 24, wherein B cells are stimulated ex vivo.
34. The method of item 24, wherein the B cells are stimulated with a Toll-like receptor (TLR) agonist.
35. The method of item 24, wherein the B cells are Breg cells.
36. The method of item 35, wherein the Breg cells express immunomodulatory cytokine IL-10.
37. The method of item 35, wherein the Breg cells comprise at least 80% CD19+ B cells.
38. The method of item 35, wherein the B cells comprise less than 10% CD138+ plasma B cells.
39. The method of item 24, wherein the B cells are formulated to be administered locally.
40. The method of item 24, wherein the B cells are formulated to be administered systemically.
41. The method of item 24, wherein the B cells are formulated to be administered intravenously, intraarterially, subcutaneously, intrathecally, or intraparenchymal.
42. The method of item 24, wherein the B cells are formulated to be administered through an intracranial cranial pressure (ICP) monitoring catheter.
43. The method of item 24, wherein the B cells are administered once daily, once weekly, twice weekly, once every 14 days, once monthly, once every two months, once every three months, once every four months, once every five months, once every six months, or once yearly.
44. The method of item 24, wherein the therapeutically effective amount comprises at least 0.5 x 107 B cells per administration.
45. The method of item 24, wherein the therapeutically effective amount comprises at least 1 x 108 B cells per administration.
46. The method of item 24, wherein the therapeutically effective amount comprises at least 2 x 108 B cells per administration.
47. The method of item 24, wherein the therapeutically effective amount comprises at least 1 x 109 B cells per administration.
48. The method of any of the aforementioned items, wherein the subject is a human.

## Claims

1. A method of treating a subject having a traumatic brain injury (TBI), comprising administering to the subject a therapeutically effective amount of isolated B cells.

2. The method of claim 1, wherein TBI results from a head injury, or a cerebral contusion.

3. The method of claim 1, wherein the subject suffers from one or more of a number of physical, cognitive, social, emotional and/or behavioral disorders.

4. The method of any one of the preceding claims, wherein allogeneic B cells, autologous B cells, or xenogeneic B cells are administered.

5. The method of any one of the preceding claims, additionally comprising administering a second therapeutic composition, in particular wherein the second therapeutic composition is an antibiotic or a corticosteroid.

6. The method of any one of the preceding claims, wherein the B cells are mature naïve B cells.

7. The method of any one of the preceding claims, wherein B cells are stimulated ex vivo, and/or wherein the B cells are stimulated with a Toll-like receptor (TLR) agonist.

8. The method of any one of the preceding claims, wherein the B cells are B_{reg} cells.

9. The method of claim 8, wherein the B_{reg} cells
- express immunomodulatory cytokine IL-10,
- comprise at least 80% CD19+ B cells, and/or
- comprise less than 10% CD138+ plasma B cells.

10. The method of any one of the preceding claims, wherein the B cells are formulated to be administered locally, or systemically, and/or wherein the B cells are formulated to be administered intravenously, intraarterially, subcutaneously, intrathecally, or intraparenchymally.

11. The method of any one of the preceding claims, wherein the B cells are formulated to be administered through an intracranial cranial pressure (ICP) monitoring catheter.

12. The method of any one of the preceding claims, wherein the B cells are administered once daily, once weekly, twice weekly, once every 14 days, once monthly, once every two months, once every three months, once every four months, once every five months, once every six months, or once yearly.

13. The method of any one of the preceding claims, wherein the therapeutically effective amount comprises at least 0.5 x 10⁷ B cells per administration, preferably at least 1 x 10⁸ B cells per administration, more preferably at least 2 x 10⁸ B cells per administration, in particular at least 1 x 10⁹ B cells per administration.

14. The method of any one of the preceding claims, wherein the subject is a human.
